# EUROPEAN PATENT SPECIFICATION

(11) **EP 3 370 528 B1**
(45) Date of publication and mention of the grant of the patent: **03.04.2024**
(21) Application number: 16860405.6
(22) Date of filing: 13.02.2016
(51) Int. Cl.: A01N 63/22, A01P 1/00, C12P 1/04, C12N 1/20, A01N 59/00, A01N 37/46, A61P 31/04, A61P 31/10, A61P 31/12

(54) **PROBIOTIC COMPOSITIONS AND METHODS REDUCING PATHOGENS IN AN ENVIRONMENT**
PROBIOTISCHE ZUSAMMENSETZUNGEN UND VERFAHREN ZUR REDUZIERUNG VON PATHOGENEN IN EINER UMGEBUNG
COMPOSITIONS PROBIOTIQUES ET PROCÉDÉS RÉDUISANT LES PATHOGÈNES DANS UN ENVIRONNEMENT

(30) Priority: 30.10.2015 US 201562248710 P
(43) Date of publication of application: 12.09.2018
(73) Proprietor: Z Probiotics Inc. Dba Z Bioscience Inc., Carlsbad, California 92009 (US)
(72) Inventor: HOLMES, Gary, Carlsbad, California 92009 (US)
(74) Representative: Vossius & Partner Patentanwälte Rechtsanwälte mbB
(86) International application number: PCT/US2016/017908
(87) International publication number: WO 2017/074485

(56) References cited:
- EP-A1- 1 228 769
- WO-A1-94/11492
- WO-A1-98/04335
- WO-A1-98/47374
- WO-A1-2009/126473
- WO-A2-2009/105845
- ES-A1- 2 520 615
- US-B2- 6 905 692
- US-B2- 8 211 849
- DATABASE WPI Week 201019 Thomson Scientific, London, GB; AN 2010-A78420 XP002789859, & CN 101 611 707 A (HAINAN LIMENGTE BIOLOGICAL PESTICIDE CO) 30 December 2009 (2009-12-30)
- DATABASE WPI Week 201515 Thomson Scientific, London, GB; AN 2015-109842 XP002789860, & CN 104 222 167 A (QINGDAO JINXIU SHUIYUAN TRADING CO LTD) 24 December 2014 (2014-12-24)
- DATABASE WPI Week 201525 Thomson Scientific, London, GB; AN 2015-21845M XP002789861, & CN 104 336 074 A (QINGDAO AIHUALONG BIOLOGICAL TECHNOLOGY) 11 February 2015 (2015-02-11)
- .: "Probiotic Heavy Duty Cleaner and Foamer - Safety Data Sheet", , 6 May 2015 (2015-05-06), pages 1-2, XP055570328, Orlando, FL, U.S.A. Retrieved from the Internet: URL:http://choiceprobiotics.com/Site/Docum ents/SP-SDS%20Heavy%20Duty%20Cleaner%20May %202015.pdf [retrieved on 2019-03-18]
- .: "AIRBIOTICS STABIOTICTM MIST PROBIOTIC ENVIRONMENT CONTROL - Safety Data Sheet", , 15 September 2015 (2015-09-15), pages 1-2, XP055570331, Hollywood, FL, U.S.A. Retrieved from the Internet: URL:http://goevomed.com/wp-content/uploads /2015/10/SDS-STABIOTIC-MIST-Final.pdf [retrieved on 2019-03-18]
- .: "CHRISAL POULTRY OPERATIONS MANUAL", , 1 November 2014 (2014-11-01), pages 1-9, XP55571491, Canada Retrieved from the Internet: URL:http://choiceprobiotics.com/Site/Docum ents/CHRISAL%20POULTRY%20OPERATIONS%20MANU AL%20ver%203.5%20Nov%202014.pdf [retrieved on 2019-03-19]
- ALBERTA VANDINI ET AL: "Hard Surface Biocontrol in Hospitals Using Microbial-Based Cleaning Products", PLOS ONE, vol. 9, no. 9, 26 September 2014 (2014-09-26), page e108598, XP055570303, DOI: 10.1371/journal.pone.0108598
- VINCENZA LA FAUCI ET AL: "An Innovative Approach to Hospital Sanitization Using Probiotics: In Vitro and Field Trials", JOURNAL OF MICROBIAL & BIOCHEMICAL TECHNOLOGY, vol. 07, no. 03, 30 May 2015 (2015-05-30), pages 160-164, XP055570294, DOI: 10.4172/1948-5948.1000198
- Sante Mazzacane ET AL: "Reduction of the Microbiological Load on Hospital Surfaces Through Probiotic-Based Cleaning Procedures: A New Strategy to Control Nosocomial Infections", Journal of Microbiology & Experimentation, 6 October 2014 (2014-10-06), page 00027, XP055570305, DOI: 10.15406/jmen.2014.01.00027 Retrieved from the Internet: URL:https://pdfs.semanticscholar.org/3fdf/ b51acd22cc0aaab38b008064a85623e251ce.pdf [retrieved on 2019-03-18]
- Sante Mazzacane ET AL: "La sanificazione delle degenze ospedaliere: nuove strategie per la riduzione delle infezioni correlate all'assistenza sanitaria", L'Ospedale, 1 February 2014 (2014-02-01), pages 8-17, XP055570343, Italy Retrieved from the Internet: URL:https://www.pchs.it/userfiles/1222/fil e/l_ospedale_2-2014.pdf [retrieved on 2019-03-18]
- .: "TESTING OF BIO-STABILIZATION TECHNIQUES USING CHRISAL PROBIOTIC PRODUCTS FOR CLEANING AND SANITIZING OF HOSPITAL WARDS", The University of Ferrara Study At the St. Anne Hospital, 13 October 2011 (2011-10-13), pages 1-17, XP055570605, Retrieved from the Internet: URL:http://purebioticsinc.com/wp-content/u ploads/2015/02/University-of-Ferrera-Year- long-St-Anne-Hospital-Study-2010-to-2011.p df [retrieved on 2019-03-18]
- Robin Temmermen (Temmerman): "Healthcare The Cleaning Revolution", , 1 November 2014 (2014-11-01), pages 1-12, XP055570622, Ontario, Canada Retrieved from the Internet: URL:http://purebioticsinc.com/wp-content/u ploads/2014/11/PIP-Healthcare-the-Cleaning -Revolution.pdf [retrieved on 2019-03-18]

## Description

### BACKGROUND

Bacteria are best known as free-living single cells, but in reality their lives are much more complex. To survive in harsh environments, many species of bacteria will band together and form a biofilm-a collection of cells held together by a tough web of fibers that offers protection from all manner of threats, including cleaning products and antibiotics. A biofilm can comprise both beneficial bacteria (probiotic bacteria) and harmful bacteria (pathogenic bacteria).

Bacterial cells form biofilms. Bacterial cells that live in biofilms can be about 500 times to 1,000 times more resistant to disinfectants and cleaning products than planktonic (free floating or suspended) bacterial cells of the same species. According to the Food and Drug Administration (FDA), the only way to remove biofilm from a surface is by harsh abrasion. Biofilm is present on virtually every surface, moist or dry.

Stronger and stronger disinfectants and antibiotics are being used in attempts to remove biofilm in attempts to defeat the pathogens (pathogenic bacteria). However, pathogens are winning; they are rapidly developing resistance to new cleaning products and antibiotics. After an area or surface is cleaned with a cleaning product, biofilms and therefore pathogens still remain.

Therefore, there is a need for compositions that can remove, or decrease the amount of biofilm on a surface. There is a need for compositions that can decrease the spread of disease in a medical setting, for example, a hospital. There is a need for compositions that can make the environment safer for humans, for example, in homes, offices, businesses, transportation, public buildings, or public spaces. There is also a need for compositions that can reduce the mortality rate of a mammal, or increase the yield of an animal, for example, livestock. In addition, there is also a need for a composition that can reduce the effects of a biological crisis, such as a cholera outbreak, once it enters a livestock population. The compositions described herein provide increased immunity, increased cellular integrity, and increased gut health in young and growing animals. The compositions described herein can be used to provide a clean environment for any livestock, whether, for example, avian, bovine, or porcine. The compositions described herein can be used to provide a clean environment for humans as well.

For nearly 60 years, United States (U.S.) producers successfully targeted subclinical animal infections with antimicrobial growth promoters (AGPs). Year-on-year, sales of active antimicrobials have increased, reaching 14,600 metric tons in 2012. However, recent legislative changes have forced producers to adopt new husbandry strategies, with the FDA and U.S. pharmaceutical companies removing "dual licenses" in 2013. The latter allowed antimicrobials to be used in medicine and agricultural growth promotion. Beginning in 2017, only non-medical antimicrobials will remain, with use in animals requiring veterinary authorization. Concerns over antimicrobial gene resistance, increasing environmental pollution levels and negative consumer opinion have driven this change. Consumers are very important in driving industry changes, as they also have great political power.

As U.S. feed manufacturers re-evaluate their options, probiotics may represent one of the best technical additives with potential to 'step-up' as replacements to antibiotics.

In hospital settings, conventional cleaning and disinfectant protocols are not effective methods to addressing the problem of biofilms. Standard hospital cleaning protocols, for example, a cleaning step using a detergent, followed by a disinfection step using bleach, quaternary ammonium, or other disinfectants, does not eliminate biofilms. This is because biofilms provide physical protection from cleaning agents making microbes less susceptible to disinfectants. Daily cleaning of an ICU with existing products does little more than support the existence and propagation of these dangerous biofilms, unless after the daily cleaning the surface is thoroughly scrubbed. Therefore, new products and methods designed specifically to defeat the biofilms must be developed.
Previously, a data sheet regarding "Probiotic Heavy Duty Cleaner and Foamer - Safety Data Sheet" has been published (http://choiccprobiotics.com/Site/Documents/SP-SDS%20Heavy%20Duty%20Cleaner%20May%20201 5.pdf)
Moreover, an innovative approach to hospital Sanitization using probiotics in *in vitro* and field trials has been described (La Fauci, et al,, Journal of Microbial & Biochemical Technology 7(3): 160-164 (2015)).
Further, the reduction of the microbiological load on hospital surfaces through probiotic-based cleaning procedures and a new strategy to control nosocomial infections has been described (Mazzacane et al., Journal of Microbiology & Experimentation 1(5): 27 (2014)).

Disclosed herein are novel compositions useful in reducing the growth of microbes, such as pathogens. These novel compositions act both short term and long term. Pathogenic bacteria are killed by the novel compositions, but surprisingly, the compositions also decrease the presence or regrowth of pathogens over time, This is in stark contrast with standard cleaning protocols (treatments) and products, wherein the concentration of pathogenic bacteria are at best only decreased a very short period of time, and then come back in full force after the treatment has worn off

### SUMMARY

The present invention relates to the embodiments as characterized in the claims. Thus, it relates, in particular to the following:
A composition for reducing a pathogen in an environment or for reducing a biofilm in an environment, comprising:
a stabilizer, at least one species of Bacillus, one or more surfactants, a proteose or an amylase, and a disinfectant,
wherein the stabilizer is propylene glycol, glycerol, an oligomer or polymer of ethylene oxide, a methoxypolyethylene glycol, 1,2- dihydroxypropane, methyl glycol or trimethyl glycol;
the disinfectant is chlorine, chlorine dioxide, hydrogen peroxide, or sodium hypochlorite; and wherein the at least one species of Bacillus is Bacillus subtilis, Bacillus licheniformis, Bacillus pumilus, Bacillus coagulans, Bacillus amyloliquefaciens, Bacillus megaterium, or Bacillus mojavensis.

A method of reducing a pathogen in an environment, comprising: treating the environment with a composition as defined aboev, wherein the environment is a surface selected from the group consisting of plastic, wood, ceramic glass, stainless steel, steel, cast iron, plaster, point, other metals, and cement; air, air space, or an aqueous solution.

A method of reducing a biofilm in an environment, comprising: treating the environment with a composition as defined above, wherein the environment is a surface selected from the group consisting of plastic, wood, ceramic, glass, stainless steel, steel, cast iron, plaster, paint, other metals, and cement; air; air space; of an aqueous solution.

Disclosed herein but not part of the claimed invention are composition for reducing a pathogen in an environment or for reducing a biofilm in an environment, comprising: a) a stabilizer and at least one species of Bacillus; or b) a stabilizer, at least one species of Bacillus, and a protease or an amylase; or c) a stabilizer, at least one species of Bacillus, and a disinfectant, or d) a stabilizer, at least one species of Bacillus, a disinfectant, and a protease or an amylase; or e) a stabilizer, at least one species of Bacillus, and one or more surfactants; or f) a stabilizer, at least one species of Bacillus, one or more surfactants, and a disinfectant; or g) a stabilizer, at least one species of Bacillus, one or more surfactants, and a protease or an amylase; or h) a stabilizer, at least one species of Bacillus, one or more surfactants, a protease or an amylase, and a disinfectant. In several different embodiments, the one or more surfactant is a total volume of 0.5% to 1.0%, 1% to 10%, 10% to 20%, 20% to 30%, 30% to 40%, 40% to 50%, 50% to 60%, 60% to 70%, 70% to 80%, 80% to 90%, or 90% to 99% of the composition. In other embodiments, the stabilizer is a total volume of 0.1% to 0.2%, 0.2% to 0.3%, 0.3% to 0.4%, 0.4% to 0.5%, 0.5% to 0.6%, 0.6% to 0,7%, 0.7% to 0.8%, 0.8% to 0.9%, 0.9% to 1.0%, 1.0% to 2.0%, 2% to 5%. 5% to 10%, 10% to 20%, 20% to 30%, 30% to 40%, or 40% to 50% of the composition. In yet other embodiments, the protease is a total volume of 0.1 % to 0.2%, 0.2% to 0.3%, 0.3% to 0.4%, 0.4% to 0.5%, 0.5% to 0.6%, 0.6% to 0,7%, 0.7% to 0.8%, 0.8% to 0.9%, 0.9% to 1.0%, 1.0% to 2.0%, 2.0% to 3.0%, 3.0% to 4.0%, 4.0% to 5.0%, 5% to 10%, 10% to 20%, 20% to 30%, 30% to 40%, or 40% to 50% of the composition.

In addition, in other embodiments, the amylase is a total volume of 0.01% to 0.02%, 0.02% to 0.03%, 0.03% to 0.04%, 0.04% to 0.05%, 0.05% to 0.06%, 0.06% to 0.07%, 0.07% to 0.08%, 0.08% to 0.09%, 0.09% to 0.1%, 0.1 % to 1.0%, 1.0% to 2.0%, 2.0% to 3.0%, 3.0% to 4.0%, 4.0% to 5.0%, 5% to 10%, 10% to 20%, 20% to 30%, 30% to 40%, or 40% to 50% of the composition. In other embodiments, the at least one species of Bacillus is a total volume of 0.01% to 0.02%, 0.02% to 0.03%, 0.03% to 0.04%, 0.04% to 0.05%, 0.05% to 0.06%, 0.06% to 0.07%, 0.07% to 0.08%, 0.08% to 0.09%, 0.09% to 1.0%, 1.0% to 2.0%, 2.0% to 3.0%, 3.0% to 4.0%, 4.0% to 5.0%, 5.0% to 6.0%, 6.0% to 7.0%, 7.0% to 8.0%, 8.0% to 9.0%, 9.0% to 10.0%, 10.0% to 15.0%, 15.0% to 20.0%, or 20.0% to 25-0 % of the composition. In addition, disclosed herein for reference only, the disinfectant is a total volume of 0.000001% to 0.00001 0%, 0.00001 % to 0,00010%, 0.0001% to 0.0010%, 0.0010% to 0.010%, 0.010% to 0.1%, 0.10% to 1.0%, 1.0% to 10% of the composition.

According to the claimed invention, the at least one species of Bacillus is Bacillus subtilis, Bacillus licheniformis, Bacillus pumilus, Bacillus coagulans, Bacillus amyloliquefaciens, Bacillus megaterium, or Bacillus mojavensis. The species of Bacillus can be any isolated species. One skilled in the art can easily test a new species to determine if it is effective in a composition of the disclosure. The compositions of the disclosure can be used to treat any surface, air, air space, a respiratory system of a mammal, a gastrointestinal tract of a mammal, or an aqueous solution. As disclosed for reference only, the stabilizer is sodium benzoate, nitrate, nitrite, citric acid, sodium nitrate, benzoic acid, sulfur dioxide, vinegar, benzalkonium chloride, benzoid acid, benzyl alcohol, paraben, tartaric acid, isothiazolinone, sulfur dioxide, propylene glycol, polyethylene glycol (PEG), glycerin, glycerol, an oligomer or polymer of ethylene oxide, a methoxypolyethylene glycol, a polyethylene oxide, a polyoxyethylene, a simple polyol, 1,2-dihydroxypropane, 1,2-propanediol, methyl glycol, trimethyl glycol, or a derivative of any of the above. As disclosed for reference only, the disinfectant is chlorine, chlorine dioxide, alcohol, hydrogen peroxide, sodium hypochlorite, an oxidizer, quaternary ammonium, chlorhexidine, glutaraldehyde, a phenol, a pine-oil, or a derivative of any of the above. As disclosed for reference only, the surfactant is a soap, a detergent, a wetting agent, an emulsifier, a foaming agent, or a dispersant. The compositions disclosed herein can be used to reduce a pathogen in an environment. As disclosed for reference only, the pathogen is a pathogenic bacterium, a virus, or a fungus. The compositions disclosed herein can also be used to reduce a biofilm. As disclosed for reference only, the biofilm comprises a pathogen and the pathogen is a pathogenic bacterium, a virus, or a fungus. In some embodiments , the compositions wherein the composition is a) present on a wipe, a sponge, or a cloth; or b) a spray, a mist, a fog, a colloidal suspension of particles, a semi-solid form, or an aerosol spray; or c) a foam. Disclosed herein are several compositions A1+, A2+, B1+, CF+, C+, CO+, AC-C, AC-L, HC+, PB Foam, A1+wipe, CO+ wipe, M+, M+2, M+2s, W+, P+, W+2, W+2s, AC-S, D+-t128, J+, D+h100, Tingle, or CPD+.

Of these compositions, only CPD+ is not according to the claimed invention as it does not comprise a disinfectant selected from chlorine, chlorine dioxide, hydrogen peroxide or sodium hypochlorite. For this reason, also embodiments mentioned in the following which make use of CPD+ are not according to the claimed invention. In some embodiments, A1+, A2+, B1+, CF+, C+, CO+, AC-C, AC-L, HC+, PB Foam, M+, M+2, M+2s, W+, P+, W+2, W+2s, AC-S, D+t128, J+, D+h100, Tingle, or CPD+ are diluted in an aqueous solution. As disclosed for reference only the reduction of a pathogen in an environment of the reduction of a biofilm in an environment is determined by measuring ATP, by measuring ATP in RLU, or by measuring cell count in CFU per area. In some embodiments, the reduction is 0.1% to 1%, 1% to 10%, 10% to 20%, 20% to 30%, 30% to 40%, 40% to 50%, 50% to 60%, 60% to 70%, 70% to 80%, 80% to 90%, or 90% to 100%. Also disclosed herein are methods of reducing a pathogen in an environment, comprising: treating the environment with any one or more of the disclosed compositions. In addition, disclosed herein are methods of reducing a biofilm in an environment, comprising: treating the environment with a composition any one or more of the disclosed compositions.

Disclosed herein are compositions for reducing a pathogen in an environment or reducing biofilm in an environment, comprising: a stabilizer, one or more surfactants, at least one species of Bacillus, and either a protease or an amylase. For example, the composition is A1+, A2+, B1+, CF-F, C+, CO+, AC-C, AC-L, HC+, PB Foam, A1+ wipe, or CO+ wipe. The composition can further comprise a disinfectant. In one embodiment, the disinfectant is chlorine.

Disclosed herein are compositions for reducing a pathogen in an environment or reducing biofilm in an environment, comprising: a stabilizer, either a protease or an amylase, and at least one species of Bacillus. For example, the composition is M+, M+2, M+2s, W+, P+, W+2, W+2s, AC-S, D+t128, J+, or D+h100. The composition can further comprise one or more surfactants. The composition can further comprise a disinfectant. In one embodiment, the disinfectant is chlorine.

Disclosed herein are compositions for reducing a pathogen in an environment or reducing biofilm in an environment, comprising: a stabilizer and at least one species of Bacillus, For example, the composition is Tingle. The composition can further comprise one more surfactants. The composition can further comprise either a protease or an amylase. The composition can further comprise a disinfectant. In one embodiment, the disinfectant is chlorine.

Disclosed herein are compositions for reducing a pathogen in an environment or reducing biofilm in an environment, comprising: one or more surfactants, a stabilizer, at least one species of Bacillus, and a disinfectant. For example, the composition is CPD+, which is not according to the claimed invention. In one embodiment, the disinfectant is chlorine.

Disclosed herein are methods of reducing a pathogen in an environment or reducing biofilm in an environment, comprising: a) treating the environment with at least one composition chosen from, A1+, A2+, B1 +, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, J+, or CPD+.

Disclosed herein are methods of reducing a pathogen on a surface or reducing biofilm on a surface, comprising: a) treating the surface with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing a pathogen in an aqueous solution or reducing biofilm in an aqueous solution, comprising: a) treating the aqueous solution, one or more times, at least one composition chosen from, A1+, A2+, B1+, CF+, C+, CO+, AC-C, AC-L, HC+, PB Foam, M+, M+2, M+2s, Tingle, W+, P+, W+2, W+2s, AC-S, D+t128, J+, D+h100, or CPD+.

Disclosed herein for reference only are methods of reducing a pathogen in an aqueous solution or reducing biofilm in an aqueous solution, comprising: a) continuously treating the aqueous solution at least one composition chosen from, A1+, A2+, B1+, CF+, C+, CO+, AC-C, AC-L, HC+, PB Foam, M+, M+2, M+2s, Tingle, W+, P+, W+2, W+2s, AC-S, D+t128, J+, D+h100, or CPD+.

Disclosed herein for reference only are methods of growing a non-human mammal, comprising: a) introducing the non-human mammal to an environment, and b) treating the environment of the non-human mammal with at least one composition chosen from, A1+, A2+, B1+, CF+, C+, CO+, AC-C, AC-L, HC+, PB Foam, A1 + wipe, CO+ wipe, M+, M+2, M+2s, Tingle, W+, P+, W+2, W+2s, AC-S, D+t128, D+h100, or CPD+.

Disclosed herein for reference only are methods of growing a non-human mammal, comprising: a) introducing the non-human mammal to an environment, b) treating the environment of the non-human mammal with a first composition, c) introducing a second composition into the respiratory system of the mammal, and d) introducing a third composition into the air of the environment, wherein steps a), b), c), and d) can occur in any order, in any combination, or all four steps can occur simultaneously, and wherein the first composition, the second composition, and the third composition are each chosen from the group consisting of A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t.128, D+h100, or CPD+.

Disclosed herein for reference only are methods of increasing growth of a non-human mammal, comprising:
a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+. In several embodiments, the non-human mammal is an avian, a bovine, or a porcine. As disclosed for reference only, the non-human mammal is a livestock. As disclosed for reference only, the increase in growth can be determined by measuring an increase in weight per unit of time. As disclosed for reference only, the increase in growth can be determined by measuring an increase in weight per unit of time. As disclosed for reference only, the increase in growth can be measured in grams per day

Disclosed herein for reference only are methods of increasing growth of a non-human mammal, comprising:
a) introducing the non-human mammal to an environment, b) treating the environment of the non-human mammal with a first composition, c) introducing a second composition into the respiratory system of the mammal, and d) introducing a third composition into the air of the environment,
wherein steps a), b), c), and d) can occur in any order, in any combination, or all four steps can occur simultaneously, and wherein the first composition, the second composition, and the third composition are each chosen from the group consisting of: A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+. As disclosed for reference only, the non-human mammal is an avian, a bovine, or a porcine. As disclosed for reference only, the non-human mammal is a livestock. As disclosed for reference only, the increase in growth can be determined by measuring an increase in weight per unit of time. As disclosed for reference only, the increase in growth can be measured in grams per day.

Disclosed herein for reference only are methods of reducing mortality of a non-human mammal, comprising:
a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, Da-t128, D+h100, or CPD+.

Disclosed herein for reference only are methods of reducing mortality of a non-human mammal, comprising:
a) introducing the non-human mammal to an environment, b) treating the environment of the non-human mammal with a first composition, c) introducing a second composition into the respiratory system of the mammal, and d) introducing a third composition into the air of the environment,
wherein steps a), b), c), and d) can occur in any order, in any combination, or all four steps can occur simultaneously, and wherein the first composition, the second composition, and the third composition are each chosen from the group consisting of: A1+, A2+, B1+, CF+, C-i-, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+

Disclosed herein for reference only are methods of treating a non-human mammal that is in contact with or at risk of being infected with a disease, comprising the steps of: a) introducing the non-human mammal to an environment, and b) treating the environment of the non-human mammal with at least one composition chosen from, A1+, A2+, B1+, CF+, C+, CO+, AC-C, AC-L, HC+, PB Foam, A1 + wipe, CO+ wipe, M+, M+2, M+2s, Tingle, W+, P+, W+2, W+2s, AC-S, D+t128, D+h100, or CPD+. As disclosed for reference only, the non-human mammal has been in contact with or is at risk of being infected with Cholera, Salmonella, Coliforms, Listeria monocytogenes, Staphylococus aureus, Methicillin-resistant Staphylococcus aureus, Vancomycin-resistant Enterococcus, Vancomycin-intermediate-Staphylococcus aureus, Carbapenem-resistant Enterobacteriaceae, or bovine respiratory disease.

Disclosed herein for reference only are methods of treating a non-human mammal that is in contact with or at risk of being infected with a disease, comprising the steps of: a) introducing the non-human mammal to an environment, b) treating the environment of the non-human mammal with a first composition, c) introducing a second composition into the respiratory system of the mammal, and d) introducing a third composition into the air of the environment, wherein steps a), b), c), and d) can occur in any order, in any combination, or all four steps can occur simultaneously, and wherein the first composition, the second composition, and the third composition are each chosen from the group consisting of: A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+. As disclosed for reference only, the non-human mammal has been in contact with or is at risk of being infected with Cholera, Salmonella, Coliforms, Listeria monocytogenes, Staphylococus aureus, Methicillin-resistant Staphylococcus aureus, Vancomycin-resistant Enterococcus, Vancomycin-intermediate-Staphylococcus aureus, Carbapenem-resistant Enterobacteriaceae, or bovine respiratory disease.

Disclosed herein are methods of reducing a pathogen in a non-human mammal, comprising:
a) introducing the non-human mammal to an environment, and b) treating the environment of the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing a pathogen in a non-human mammal, comprising:
a) introducing the non-human mammal to an environment, b) treating the environment of the non-human mammal with a first composition, c) introducing a second composition into the respiratory system of the mammal, and d) introducing a third composition into the air of the environment,
wherein steps a), b), c), and d) can occur in any order, in any combination, or all four steps can occur simultaneously, and wherein the first composition, the second composition, and the third composition are each chosen from the group consisting of: A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing formation of biofilm in an environment, comprising: a) treating the environment with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, J+, or CPD+.

Disclosed herein for reference only are methods of increasing efficiency of a heat exchanger or a cooling tower, comprising: a) obtaining an aqueous solution, b) treating the aqueous solution with at least one composition chosen from A1+, A2+, Bl-+-, CF+, C+, CO+, AC-C, AC-L, HC+, M+, M+2, M+2s, W+, P+, W+2, W+2s, AC-S, c) running the aqueous solution through the piping and fluid lines of the heat exchanger or cooling tower, d) treating the surface of coil fins and tubing with at least one composition chosen from, A1+, A2+, B1+, CF+, C+, CO+, AC-C, AC-L, HC+, M+, M+2, M+2s, W+, P+, W+2, W+2s, AC-S, and e) treating the cooling tower heat exchange panels, blankets, and fill with at least one composition chosen from, A1+, A2+, B1+, CF+, C+, CO+, AC-C, AC-L, HC+, M+, M+2, M+2s, W+, P+, W+2, W+2s, AC-S.

Disclosed herein for reference only are methods of increasing the sensitivity of a non-human mammal to an antibiotic, comprising: a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of increasing the sensitivity of a non-human mammal to an antibiotic, comprising: a) introducing the non-human mammal to an environment, b) treating the environment of the non-human mammal with a first composition, c) introducing a second composition into the respiratory system of the mammal, and d) introducing a third composition into the air of the environment, wherein steps a), b), c), and d) can occur in any order, in any combination, or all four steps can occur simultaneously, and wherein the first composition, the second composition, and the third composition are each chosen from the group consisting of: A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of increasing gastrointestinal health of a non-human mammal to an antiobiotic, comprising: a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle. W+, P+, W+2, W+2s, AC-S, D+t128, D+h100, or CPD+.

Disclosed herein for reference only are methods of increasing gastrointestinal health of a non-human mammal to an antibiotic, comprising: a) introducing the non-human mammal to an environment, b) treating the environment of the non-human mammal with a first composition, c) introducing a second composition into the respiratory system of the mammal, and d) introducing a third composition into the air of the environment, wherein steps a), b), c), and d) can occur in any order, in any combination, or all four steps can occur simultaneously, and wherein the first composition, the second composition, and the third composition are each chosen from the group consisting of: A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing laminitis in a non-human mammal, comprising:
a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing scours in a non-human mammal, comprising: a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing Mastitis in a non-human mammal, comprising:
a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing somatic cell count in a non-human mammal comprising: a) treating the non-human mammal with at least one composition chosen from, A1+, A2+, B1+, CF+, C+, CO+, AC-C, AC-L, HC+, PB Foam, A1+ wipe, CO+ wipe, M+, M+2, M+2s, Tingle, W+, P+, W+2, W+2s, AC-S, D+t128, D+h100, or CPD+.

Disclosed herein for reference only are methods of reducing a pathogen from an environment of a fish or reducing biofilm from an environment of a fish, comprising: a) obtaining an aqueous solution, b) treating the aqueous solution, one or more times, at least one composition chosen from, A1+, A2+, B1+, CF+, C+, CO+, AC-C, AC-L, HC+, PB Foam, M+, M+2, M+2s, Tingle, W+, P+, W+2, W+2s, AC-S, D+t128, D+h100, or CPD+, and c) placing a fish into the aqueous solution, wherein the fish can be placed into the aqueous solution prior to of after treating the aqueous solution with the at least one composition.

In any of the compositions disclosed herein, one or more surfactant is a total volume of 0.5% to 1.0%, 1% to 10%, 10% to 20%, 20% to 30%, 30% to 40%, 40% to 50%, 50% to 60%, 60% to 70%, 70% to 80%, 80% to 90%, or 90% to 99% of the composition.

In any of the compositions disclosed herein, the stabilizer is a total volume of 0.1% to 0.2%, 0.2% to 0.3%, 0.3% to 0.4%, 0.4% to 0.5%, 0.5% to 0.6%, 0.6% to 0,7%, 0.7% to 0.8%, 0.8% to 0.9%, 0.9% to 1.0%, 1.0% to 2.0%, 2% to 5%, 5% to 10%, 10% to 20%, 20% to 30%, 30% to 40%, or 40% to 50% of the composition.

In any of the compositions disclosed herein, the protease is a total volume of 0.1% to 0.2%, 0.2% to 0.3%, 0.3% to 0.4%, 0.4% to 0.5%, 0.5% to 0.6%, 0.6% to 0,7%, 0.7% to 0.8%, 0.8% to 0.9%, 0.9% to 1.0%, 1.0% to 2.0%, 2.0% to 3.0%, 3.0% to 4.0%, or 4.0% to 5.0% of the composition.

In any of the compositions disclosed herein, the amylase is a total volume of 0.01% to 0.02%, 0.02% to 0.03%, 0.03% to 0.04%, 0.04% to 0.05%, 0.05% to 0.06%, 0.06% to 0.07%, 0.07% to 0.08%, 0.08% to 0.09%, 0.09% to 0.1%, 0.1% to 1.0%, 1.0% to 2.0%, 2.0% to 3.0%, 3.0% to 4.0%, or 4.0% to 5.0% of the composition.

In any of the compositions disclosed herein, the at least one species of Bacillus is a total volume of 0.01% to 0.02%, 0.02% to 0.03%, 0.03% to 0.04%, 0.04% to 0.05%, 0.05% to 0.06%, 0.06% to 0.07%, 0.07% to 0.08%, 0.08% to 0.09%, 0.09% to 1.0%, 1.0% to 2.0%, 2.0% to 3.0%, 3.0% to 4.0%, 4.0% to 5.0%, 5.0% to 6.0%, 6.0% to 7.0%, 7.0% to 8.01%, 8.0% to 9.0%, 9.0% to 10.0%, 10.0% to 15.0%, 15.0% to 20.0%, or 20.0% to 25.0% of the composition.

Any of the compositions disclosed herein can be diluted in any aqueous solution. A1+ can be diluted from about 1:50 to about 1:128, from about 1:2 to about 1:200, or about 1:128. A2+ can be diluted from about 1:32 to about 1:75, from about 1:10 to about 1:128, or about 1:64. B1+ can be diluted from about 1:50 to about 1:128, from about 1:2 to about 1:128, or about 1:128. CF+ can be diluted from about 1:50 to about 1:128, from about 1:2 to about 1:256, or about 1:128. C+ can be diluted from about 1:10 to about 1:40, from about 1:5 to about 1:128, from about 1:1 to about 1:128, or about 1:30. CO+ can be diluted from about 1:5 to about 1:40, from about 1:2 to about 1:64, from about 1:1 to about 1:128, or about 1:30. AC-C can be diluted from about 1:5 to about 1:40, from about 1:2 to about 1:128, or about 1:30. AC-L can be diluted from about 1:32 to about 1:75, from about 1:5 to about 1:128, or about 1:64. HC+ can be diluted from about 1:1 to about 1:10 or from about 1:1 to about 1:50. PB Foam can be diluted from about 1:2 to about 1:10, from about 1:1 to about 1:20, or about 1:10. M+ can be diluted from about 1:1 to about 1:2, from about 1:1 to about 1:50, or about 1:2. M+2 can be diluted from about 1:2 to about 1:4, from about 1:0.1 to about 1:100, or about 1:4. M+2s can be diluted from about 1:2 to about 1:4, from about 1:0.1 to about 1:100, or about 1:4. W+ can be diluted. from about 1:1,500 to about 1:20,000, from about 1:1 to about 1:50,000, from about 1:100 to about 1:100,000, or about 1:10,000. P+ can be diluted from about 1:2,500 to about 1:20,000, from about 1:1,000 to about 1:50,000, from about 1:1 to about 1:100,000, or about 1:5,000. W+2 can be diluted from about 1:3,000 to about 1:40,000, from about 1:1, to about 1:100,000, or about 1:20,000. W+2s can be diluted from about 1:3,000 to about 1:40,000, from about 1:1 to about 1:100,000, or about 1:20,000. AC-S can be diluted from about 1:1 to about 1:2, from about 1:1 to about 1:50, or about 1:2. D+t128 wipe can be diluted from about 1:100 to about 1:150, from about 1:10 to about 1:256, or about 1:128. D+t128 can be diluted from about 1:100 to about 1:150, from about 1:10 to about 1:256, or about 1:128. D+h100 can be diluted from about 1:75 to about 1:128, from about 1:10 to about 1:256, or about 1:100. J+ can be diluted from about 1:5 to about 1:200 or from about 1:5 to about 1:20. Tingle can be diluted from about 1:5 to about 1:20, from about 1:1 to about 1:100, or about 1:20.

In any of the compositions disclosed herein, a stabilizer can be, for example, sodium benzoate, nitrate, nitrite, citric acid, sodium nitrate, benzoic acid, sulfur dioxide, vinegar, benzalkonium chloride, benzoic acid, benzyl alcohol, paraben, tartaric acid, isothiazolinone, sulfur dioxide, or a derivative of any one of the above. A stabilizer can be, for example, propylene glycol, polyethylene glycol (PEG), glycerin, glycerol, an oligomer or polymer of ethylene oxide, a methoxypolyethylene glycol, a polyethylene oxide, a polyoxyethylene, a simple polyol, 1,2-dihydroxypropane, 1,2-propanediol, methyl glycol, trimethyl glycol, or a derivative of any of the above. According to the claimed invention the stabilizer is selected from propylene glycol, glycerol, an oligomer or polymer of ethylene oxide, a methoxypolyethylene glycol, 1,2-dihydroxypropane, methyl glycol or trimethyl glycol.

A composition of the disclosure can have, for example, a pH of from about 4.5 to about 9.5, about 5 to about 9, about 3.5 to about 10.5, about 8.75 +/- 0.25, or about 8.25 +/- 0.25.

A composition according to the invention comprises a Bacillus species selected from Bacillus subtilis, Bacillus lichenifomiis, Bacillus pumilus, Bacillus coagulans, Bacillus amyloliquefaciens, Bacillus megaterium, or Bacillus mojavensis.

A pathogen that can be reduced by any of the disclosed compositions can be, for example, a pathogenic bacterium, a virus, or a fungus. For example, a pathogen can be bovine herpesvirus 1, 2 (infections rhinotracheitis), bovine viral diarrhoea virus (pestivirus), parainfluenza virus (PI 3), bovine respiratory syncytial virus, Mannheimia haemolytica, Pasteurella multocida, Histophilus somnus, actinomyces, actinobacillus, mycoplasma, Dichelobacter nodosus, or Fusobacterium necrophorum. A pathogen can be a pathogenic bacteria, for example, a species of *Legionella*, *Listeria monocytogenes, Staphylococcus aureus,* coliform, a species of *Mycobacterium,* a species of *E. coli,* a species of *Enterococcus,* a species of *Streptococcus,* a species of *Salmonella*, a species of *Staphylococcus,* a species of *Cryptosporidium,* a species of *Pseudomonas,* a species of *Candida,* or a Nematode worm. A pathogenic bacteria can be, for example, Pseudomonas aeruginosa, Clostridium difficile (C. Diff), Neisseria gonorrhacae, Carbapenem-resistant Enterobacteriaceae (CRE), Drug-resistant Streptococcus pneumoniae, Drug-resistant Carnpylobacateria, Drug-resistant non-typhoidal Salmonella, Methicillin-resistant Staphylococcus aureus (MRSA), Drug-resistant Shigella, Extended-spectrum Enterobacteriaceae (ESBL), Vancomycin-resistant Enterococcus (VRE), Multidrug-resistant Acinetobacteria, Multidrug-resistant Pseudomonas aeruginosa, Drug-resistant Salmonella serotype Typhi, Fluconazole-resistant Candida, Drug-resistant Tuberculosis, Clindamycin-resistant Group B Streptococcus, Erythromycin-resistant Group A Streptococcus, or Vancomycin-resistant Staphylococcus aureus. A pathogen can be, for example, Salmonella, Staphyloccus aureus, Coliform, Campylobacteria, causes digital or interdigital dermititis, or causes Mastitis.

Any of the disclosed compositions can be present on a wipe, a sponge, or a cloth. Any of the disclosed compositions can be a spray, a mist, a colloidal suspension of particles, a semi-solid form, or an aerosol spray. In one embodiment, a composition can be a foam.

In any of the compositions or methods disclosed herein, an environment that can be cleaned by any of the disclosed compositions can be, for example, a surface, air, air space or water supply.

In any of the methods disclosed herein, a reduction in a pathogen can be determined by measuring ATP, or by measuring ATP in RLU. A reduction in a pathogen can be determined by measuring cell count in CFU per area. A reduction in a pathogen can be 0.1 to 1%, 1% to 10%, 10% to 20%, 20% to 30%, 30% to 40%, 40% to 50%, 50% to 60%, 60% to 70%, 70% to 80%, 80% to 90%, or 90% to 100%.

In any of the methods disclosed herein, a reduction in biofilm is determined by measuring ATP, or by measuring ATP in RLU. A reduction in biofilm is determined by measuring cell count in CFU per area. A reduction in biofilm can be 0.1 to 1%, 1% to 10%, 10% to 20%, 20% to 30%, 30% to 40%, 40% to 50%, 50% to 60%, 60% to 70%, 70% to 80%, 80% to 90%, or 90% to 100%.

In any of the methods disclosed herein, a non-human or human mammal can be treated with any of the disclosed compositions. A treatment can occur 1 to 2 times, 2 to 5 times, 5 to 10 times, 10 to 100 times, more than 100 times, more than 1,000 times, more than 10,000 times, or throughout the life of the non-human or human mammal. Such method of treatment is not part of the claimed invention.

A non-human mammal can be, for example, an avian, a bovine, or a porcine. A non-human mammal can be, for example, a livestock.

A composition can be introduced into the environment of a non-human or human mammal. A composition can be introduced by adding the composition to an aqueous solution that is consumed by the non-human or human mammal. As disclosed for reference only, the introduction is repeated two or more times. As disclosed for reference only, the introduction is continuous. As disclosed for reference only, the introduction is repeated throughout the life of the non-human or human mammal. As disclosed for reference only, the introduction is intermittent throughout the life of the non-human or human mammal. A composition can be introduced into the environment of a non-human or human mammal by misting, fogging, spraying, or aerosol. In no case throughout the description ought a method or a use involving the treatment of a human or an animal to be regarded as encompassed by the claimed invention.

### BRIEF DESCRIPTION OF THE DRAWINGS

These and other features, aspects, and advantages of the present invention will become better understood with regard to the following description, appended claims and accompanying figures where:
FIG. 1 shows biofilm formation.
FIG. 2 shows a biofilm "shelter" comprising a biofilm binding matrix and microorganisms that are living in the biofilm.
FIG. 3 shows the presence of biofilm in numerous locations and on numerous surfaces.
FIG. 4 shows removal of biofilm from an exemplary surface.
FIG. 5 shows a comparison of cleaning a surface with a disinfectant versus a probiotic composition of the disclosure.
FIG. 6 shows the efficacy of M+ to treat Listeria Monocytogenes.
FIG. 7 shows removal of biofilm from stairs.
FIG. 8 shows the efficacy of CF+ compared to a commercially available cleaner.
FIG. 9 shows a comparison of poult manure droppings.
FIG. 10 shows the efficacy of several compositions of the disclosure in a hatchery.
FIG. 11 shows the efficacy of C+ in a "boot" test.
FIG. 12 shows the efficacy of C+ to clean internal surfaces of water lines.
FIG. 13 shows the efficacy of W+ on turkey mortality.
FIG. 14 shows the efficacy of W+ on turkey weight.
FIG. 15 shows the effect of W+ on swine gut health.
FIG. 16 shows the effect of CO+ on surfaces of a processing plant.
FIG. 17 shows the effect of CO+ on surfaces of a processing plant.
FIG. 18 shows the date of FIG. 17.
FIG. 19 shows bacterial build-up beneath the surface of a biofilm.
FIG. 20 shows stages in the formation of a biofilm.
FIG. 21 shows the effect of D+t128 on reducing the presence of Mastitis.
FIG. 22 shows the efficacy of D+h100 to reduce hairy wart and foot rot infection in cows.
FIG. 23 shows the efficacy of W+ on turkey mortality.
FIG. 24 shows the efficacy of AC-C and AC-S on HVAC coils.

### DETAILED DESCRIPTION

The following detailed description is provided to aid those skilled in the art in practicing the present invention.

As used in this disclosure and the appended claims, the singular forms "a", "an" and "the" include a plural reference unless the context clearly dictates otherwise. As used in this disclosure and the appended claims, the term "or" can be singular or inclusive. For example, A or B, can be A and B.

Disclosed herein are novel compositions (products) that act on biofilms. The probiotics present in the composition divide, and thrive, out competing pathogenic bacteria for nutrients and resources. This results in decreased numbers of pathogenic bacteria, increased numbers of probiotic bacteria, and the breaking down and/or removal of the biofilm.

### ADVANTAGES OF THE COMPOSITIONS OF THE DISCLOSURE

Commonly used disinfectants (sanitizers), for example, bleach, quaternary ammonium, chlorhexidine, glutaraldehyde, phenols, and any other disinfectants known to one of skill in the art, kill both beneficial and pathogenic bacteria. The resistance of a disinfectant can be passed down through generations of bacteria. Any effect of disinfectants on the reduction of biofilms is short term. The use of disinfectants is harmful to both humans and animals, and to the environment. Surfaces that are cleaned with disinfectants often corrode over repeated cleaning with the disinfectant, and disinfectants are expensive.

The novel compositions (products) are not hazardous upon inhalation, skin contact, or eye contact, and have no significant hazards when ingested by humans or animals. This is in contrast with existing disinfectants (sanitizers) and cleaners that can have serious negative effects on both animals and humans that come in contact with them. The novel compositions disclosed herein are environmentally safe, biologically degradable, non-toxic, non-flammable, and non-volatile.

The compositions disclosed herein are an attractive alternative to the use of antibiotics in that the use of the compositions does not result in the development and spread of resistant strains of bacteria.

The compositions disclosed herein can be used by any livestock farmer to promote the health of its animals. For example, use of the compositions can balance intestinal microflora present in the gut of the animal and/or inhibit growth of pathogens in the animal or the animal's drinking water. A healthier animal results in a higher quality meat product, reduced use of and presence of antibiotics, reduced mortality, and reduced veterinarian bills. The compositions have been registered for use with Organic Livestock, and certified Green and Eco-Friendly. Such use in or on animals is not part of the claimed invention.

The compositions disclosed herein can be used to reduce the presence of pathogens in an animal's environment. For example, the compositions can reduce or eliminate the presence of Salmonella, Staphylococcus, Coliforms, Campylobacter, or any other pathogenic bacteria, from an animal's environment.

The compositions disclosed herein, can increase productivity in the livestock industry, and do so without the insurgence of a large number of new pathogens that are resistant to antibiotics. Livestock farmers do not have to be dependent on hazardous chemicals that result in costly cleanup are harmful to their livestock and dangerous to the farmer, and results in negative public relations. The livestock will have improved health and increased growth performance without the use of antibiotics. The compositions disclosed herein can be used to reduce or eliminate the presence of biofilm in an animal's water source, therefore reducing the negative effects of pathogenic bacteria.

Animals, in which they themselves and/or their environment, are treated with the compositions disclosed herein display increased weight gain, improved feed conversion, lower mortality, resistance to viral challenges, and better overall health. The result of controlling the presence of pathogens in the animals' environment is healthier and stronger animals and reduced mortality. The treatment of animals is not part of the claimed invention.

The compositions described herein can also be used to reduce, eliminate, or prevent the presence of biofilm on any surface (hard or soft), space, air space, or water source, public or private. For example, a surface (hard or soft), space, air space, or water source, can be present in a medical setting (for example, a hospital, a doctor's office, a dental office, any medical office, a contact lense, or a medical device). For example, a surface (hard or soft), space, air space, or water source, can be present in a home, office, business, public space, or public office. For example, a surface (hard or soft), space, air space, or water source, can be present in an airport, train, bus, plane, grocery store, or restaurant. Hard and soft surfaces within the scope of the methods of the invention do not include surfaces of humans and or animals.

### OTHER PRODUCTS USED TO CLEAN, DISINFECT, AND SANITIZE

Traditional cleaners, sanitizers, and disinfectants are not effective in removing biofilm, which is the shelter for pathogenic bacteria.

Commonly used cleaning products include: chlorhexidine, glutaraldehyde, pine-oil derivatives, soaps, and any other cleaning products known to one of skill in the art.

The terms "disinfectant" and "sanitizer" can be used interchangeably throughout the disclosure. Exemplary disinfectants and sanitizers are: bleach, oxidizers, chlorine, chlorhexidine, glutaraldehyde, phenols, pine-oil derivatives, hydrogen peroxide, biocides, chloroxylenol ('Dettol'), cresol, hexaehlorophane, antiseptics, germicides, sterilizers, cleansers, decontaminants, fumigants, vinegar, chlorine, iodine, quaternary ammonium, and any other disinfectants or sanitizers known to one of skill in the art. Disinfectants used in the composition (s) according to the invention are chlorine, chlorine dioxide, hydrogen peroxide or sodium hypochlorite.

**TABLE 1: PRODUCT DESCRIPTIONS**

| **Product** | **General Non-limiting Description** |
|---|---|
| C+ | C+ is a composition used to clean, for example, hatcheries, growing barns, or any fouled animal housing. Immediately after cleaning a surface, a healthy and stable microbial community is created on the surface by safe microorganisms. C+ is long lasting and cleans a surface, often without the need for scrubbing. After rinsing C+ off of the surface, a barrier to pathogen colonization is formed by a layer of benevolent microorganisms that remain on the surface. The microorganisms will attack any biofilm present on the surface and within a short period break down and eliminate the biofilm shelter comprising pathogens, fungi, and viruses. C+ can be added to the water supply of an animal. |
| M+ | M+ can be applied to any surface. M+ can be applied to a surface after it has been cleaned with, for example, C+. M+ can be applied by, for example, by spraying. |
| W+ | W+ is a water supplement for use in livestock drinking water. W+ simultaneously provides a health supplement in the drinking water while controlling the presence of biofilm in the water lines. W+ can help maintain normal intestinal microflora. The metabolism in an animal's gut is altered such that there is reduced pathogenic activity and less ammonia production. The animals are therefore able to feed more effectively and improve their digestion. W+ stimulates the animal's immune system and can neutralize entero-toxins that are present in the animal's digestive system. |

**TABLE 2: PRODUCT STOCK SOLUTIONS IN PERCENTAGE OF TOTAL VOLUME**

| **Part No.** | **Ingredient** | **A1+** | **A2+** | **B1+** | **CF+** |
|---|---|---|---|---|---|
| - | Water | 45.8 | 0.44 | 45.0 | 44.8 |
| P1 | Chlorine | 0.002 | 0.002 | 0.002 | 0.002 |
| P50 | EDTA | 0.5 | 0.5 | 0.5 | 0.5 |
| A1 | Glycolic Acid | 0.2 | 0.2 | 0.2 | 0.2 |
| F1 | Anti Foam | 0.05 | | 0.8 | 1 |
| S1 | Surfactant | 50.0 | 47.7 | 50.0 | 50.0 |
| S4 | Surfactant | | 47.7 | | |
| O1 | Odorant | 1 | 1 | 1 | 1 |
| E1 | Protease | 1.05 | 1.05 | 1.05 | 1.05 |
| E2 | B. Amylase | 0.45 | 0.45 | 0.45 | 0.45 |
| | Propylene Glycol | 0.9 | 0.9 | 0.9 | 0.9 |
| | B. subtilis | 0.018 | 0.018 | 0.018 | 0.018 |
| | B. licheniformis | 0.018 | 0.018 | 0.018 | 0.018 |
| | B. pumilus | 0.018 | 0.018 | 0.018 | 0.018 |

| **Part No.** | **Ingredient** | **C+** | **CO+** | **M+** | **W+** |
|---|---|---|---|---|---|
| - | Water | 26.7 | 1.8 | 98.9 | 96.4 |
| P1 | Chlorine | 002 | .002 | .002 | .002 |
| P50 | EDTA | 0.5 | | | |
| A1 | Glycolic Acid | 0.2 | 0.2 | | |
| F1 | Anti Foam | 0.1 | 0.05 | 0.01 | 0.01 |
| S1 | Surfactant | 70.0 | | 0.1 | 0.1 |
| S4 | Surfactant | | 95.45 | | |
| O1 | Odorant | | | | 1 |
| E1 | Protease | 1.05 | 1.05 | 0.25 to 5.0 | 1.05 |
| E2 | B. Amylase | 0.45 | 0.45 | 0.25 to 4.0 | 0.45 |
| | Propylene Glycol | .9 | .9 | .9 | .9 |
| | B. subtilis | .018 | .018 | .018 | .03 |
| | B. licheniformis | .018 | .018 | .018 | .03 |
| | B. pumilus | .018 | .018 | .018 | .03 |
| | B. coagulans | | | | .03 |

| **Part No.** | **Ingredient** | **P+** | **M+2** | **M+2s** | **W+2** |
|---|---|---|---|---|---|
| - | 96.4 | 96.4 | 98.9 | 98.9 | 95.8 |
| P1 | Chlorine | .002 | .002 | .002 | .002 |
| P50 | EDTA | | | | |
| A1 | Glycolic Acid | | | | |
| F1 | Anti Foam | 0.01 | 0.01 | 0.01 | 0.01 |
| S1 | Surfactant | 0.1 | 0.1 | 0.1 | 0.1 |
| S4 | Surfactant | | | | |
| O1 | Odorant | 1.0 | | | |
| E1 | Protease | 1.05 | 0.25 to 5.0 | 0.25 to 5.0 | 2.1 |
| E2 | B. Amylase | 0.45 | 0.25 to 4.0 | 0.25 to 4.0 | 0.9 |
| | Propylene Glycol | 0.9 | 0.9 | 0.9 | 0.9 |
| | B. subtilis | 0.03 | 0.24 | 0.36 | 0.036 |
| | B. licheniformis | 0.03 | 0.24 | 0.36 | 0.036 |
| | B. pumilus | 0.03 | 0.24 | 0.36 | 0.036 |
| | B. coagulans | | | | 0.036 |

| **Part No.** | **Ingredient** | **W+2s** | **AC-C** | **AC-S** | **AC-L** |
|---|---|---|---|---|---|
| - | Water | 95.7 | 25.2 | 96.4 | 40.5 |
| P1 | Chlorine | 0.002 | 0.002 | 0.002 | 0.002 |
| P50 | EDTA | | 0.5 | | 4.0 |
| A1 | Glycolic Acid | | 0.2 | | 0.3 |
| F1 | Anti Foam | 0.01 | 0.1 | 0.01 | 1.2 |
| S1 | Surfactant | 0.1 | 70.0 | 0.1 | 50.0 |
| S4 | Surfactant | | | | |
| O1 | Odorant | | | 1.0 | |
| E1 | Protease | 2.1 | 2.1 | 1.05 | 2.1 |
| E2 | B. Amylase | 0.9 | 0.9 | 0.45 | 0.9 |
| | Propylene Glycol | 0.9 | 0.9 | 0.9 | 0.9 |
| | B. subtilis | 0.06 | 0.018 | 0.03 | 0.04 |
| | B. licheniformis | 0.06 | 0.018 | 0.03 | 0.04 |
| | B. pumilus | 0.06 | 0.018 | 0.03 | 0.04 |
| | B. coagulans | 0.06 | | | |

| **Part No.** | **Ingredient** | **CPD+** 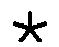 | **D+t128** | **HC+** |
|---|---|---|---|---|
| - | Water | | 95.8 | 1.7 |
| P1 | Chlorine | | 0.002 | 0.002 |
| P50 | EDTA | | | |
| A1 | Glycolic Acid | | | |
| F1 | Anti Foam | | 0.5 | .05 |
| S1 | Surfactant | 2.0 | 0.25 | |
| S4 | Surfactant | | | 95.8 |
| O1 | Odorant | | 1.0 | |
| E1 | Protease | | 1.05 | 1.05 |
| E2 | B. Amylase | | 0.45 | 0.45 |
| | Propylene Glycol | 0.9 | 0.9 | 0.9 |
| Alcohol | Alcohol 70% | 97.0 | | |
| Colorant | Color Mix | 0.025 | | |
| | B. subtilis | 0.03 | 0.16 | 0.027 |
| | B. licheniformis | 0.03 | 0.16 | 0.027 |
| | B. pumilus | 0.03 | 0.16 | 0.027 |

| | | | | |
|---|---|---|---|---|
| * CPD+ is not according to the invention | | | | |

| **Part No.** | **Ingredient** | **D+h100** | **Tingle** | **PB Foam** | **A1+ wipe** |
|---|---|---|---|---|---|
| - | Water | 95.8 | 94.0 | 10.1 | 97.5 |
| P1 | Chlorine | 0.002 | 0.002 | 0.002 | 0.002 |
| P50 | EDTA | | 1.0 | | 0.01 |
| A1 | Glycolic Acid | | | | 0.2 |
| F1 | Anti Foam | 0.05 | 0.01 | | |
| S1 | Surfactant | 0.25 | 0.006 | | 0.5 |
| S4 | Surfactant | | | 75.0 | |
| O1 | Odorant | 1.0 | 2.0 | 4.0 | 1.0 |
| E1 | Protease | 1.05 | 0.15 | | 0.15 |
| E2 | B. Amylase | 0.45 | 0.075 | | 0.075 |
| | Propylene Glycol | 0.9 | 0.9 | 0.5 | 0.5 |
| Colorant | Color Mix | | 0.025 | | |
| Glycerin | | | | 10.0 | |
| | B. subtilis | 0.16 | 0.03 | 0.01 | 0.01 |
| | B. licheniformis | 0.16 | 0.03 | 0.01 | 0.01 |
| | B. pumilus | 0.16 | 0.03 | 0.01 | 0.01 |

| **Part No.** | **Ingredient** | **CO+ wipe** |
|---|---|---|
| - | Water | 94.3 |
| P1 | Chlorine | 0.002 |
| P50 | EDTA | |
| A1 | Glycolic Acid | |
| F1 | Anti Foam | |
| S1 | Surfactant | |
| S4 | Surfactant | 2.9 |
| O1 | Odorant | 2.0 |
| E1 | Protease | 0.15 |
| E2 | B. Amylase | 0.08 |
| | Propylene Glycol | 0.5 |
| | B. subtilis | 0.01 |
| | B. licheniformis | 0.01 |
| | B. pumilus | 0.01 |

| **Part No.** | **Ingredient** | **J+** |
|---|---|---|
| - | Water | 93.7 |
| P1 | Chlorine | 0.002 |
| P50 | EDTA | |
| A1 | Glycolic Acid | |
| F1 | Anti Foam | |
| S1 | Surfactant | |
| S4 | Surfactant | |
| O1 | Odorant | |
| E1 | Protease | 2.1 |
| E2 | B. Amylase | 0.9 |
| | Propylene Glycol | 0.9 |
| | B. subtilis | 0.06 |
| | B. licheniformis | 0.06 |
| | B. pumilus | 0.06 |
| | B. coagulans | 0.06 |

**TABLE 3: DESCRIPTION OF INGREDIENTS**

| **Part No.** | **Ingredient** | **Description or Source of Ingredient** |
|---|---|---|
| | | **Product Information** |
| - | Water | Demineralize water |
| | | Remove chlorine |
| | | Pass water through UV filter |
| P1 | Chlorine | Clorox 8.5% sodium hypochlorite |
| P50 | EDTA | Dow Chemical |
| | | Powder form |
| A1 | Glycolic Acid | Liquid form |
| | | Organic grade |
| | | ChemPoint |
| F1 | Anti Foam | Anti Foam diluted 1:10 prior to use in compositions |
| | | New London Chemical, C2020-A. silicon defoamer |
| S1 | Surfactant | Concentrated Liquid Surfactant |
| | | Winsol Laboratories, Inc. |
| S4 | Surfactant | Liquid Nanotechnology Surfactant |
| O1 | Odorant | Essential oil-spearmint |
| | | Essential oil-vanilla |
| | | Odorant diluted prior to adding to compositions |
| | | Creative Fragrances |
| E1 | Protease | Bio-Cat Company |
| E2 | B. Amylase | Bio-Cat Company |
| PG | propylene glycol | Huntsman Chemical |
| | Glycerin | Organic |
| | | JEDWARDS Int'l. |
| | Colorant | Color Mix (*see* **Table. 6**) |
| | Alcohol | at least 70% or greater |
| | | Carolina Biological |

**TABLE 4: ANTIFOAM STOCK SOLUTION**

| **F1** | *5 gallon volume | % of total volume |
|---|---|---|
| | 0.34432 mL Chlorine | 0.002 |
| | 4.5 gallon Water | 90% |
| | 0.5 gallon C-2020A silicone deformer concentrate (Anti Foam) | 10% |

**TABLE 5: ODORANT STOCK SOLUTION**

| **O1** | *5 gallon volume | % of total volume |
|---|---|---|
| | 0.34432 mL Chlorine | 0.002 |
| | 4.5 gallon Water | 90% |
| | 0.5 gallon C-2020A silicone deformer concentrate (Anti Foam) | 10% |

**TABLE 6: Colorant Stock Solution**

| **Color Mix** | 1 gallon volume | % of total volume |
|---|---|---|
| | 0.34432 mL Chlorine | 0.002 |
| | 1 gallon water1, SC-4nx2, "C" Conc3 | 98.7% |
| | 50 grams colored dye powder, color can be yellow, green, red or blue | 1.3% |

Each of the compositions disclosed herein, for example, A1+, A2+, B1+, CF+, C+, CO+, AC-C, AC-L, HC+, PB Foam, A1+ wipe, CO+ wipe, M+, M+2, M+2s, W+, P+, W+2, W+2s, AC-S, D+t128, J+, D+h100, Tingle, and CPD+, comprise several core compounds. For example, a stabilizer, at least one species of Bacillus, and a disinfectant. The percent total volume of one compound of the composition, for example, a stabilizer, can vary. If the percent total volume of a stabilizer is, for example, 40% of the total volume, the percent volume of the remaining compounds, such as the at least one species of Bacillus and the disinfectant can easily be calculated and adjusted by one of skill in the art to equal, for example, 60% or 100%. If 60% of the total volume is a stabilizer, at least one species of Bacillus, and a disinfectant, then the remaining 40% can be, for example, any aqueous solution, or alternatively, water and other compounds, such as EDTA or Anti Foam. In addition to the core compounds in each composition, additional compounds can be added, including additional enzymes.

### DISINFECTANTS

Chlorine is used as a disinfectant in the compositions disclosed herein. One or more disinfectants can be used in the compositions disclosed herein. Any disinfectant known to one of skill in the art, such as those disclosed herein, can be used in the compositions of the disclosure. For example, a disinfectant can be chlorine dioxide, alcohol, or hydrogen peroxide. According to the claimed invention, the disinfectant is selected from chlorine, chlorine dioxide, hydrogen peroxide or sodium hypochlorite.

### A STABILIZER

Polyethylene glycol (PEG), or any humectant can be used as a stabilizer in the compositions disclosed herein. One of skill in the art would know how to determine the effective amount of the stabilizer to add to a stock solution or to a diluted stock solution. Exemplary stabilizers include, sodium benzoate, nitrate, nitrite, citric acid, sodium nitrate, benzoic acid, sulfur dioxide, vinegar, benzalkonium chloride, benzoic acid, benzyl alcohol, paraben, tartaric acid, isothiazolinone, sulfur dioxide, or a derivative of any one of the above.

Propylene glycol can also be used as a stabilizer in the compositions disclosed herein. However, propylene glycol, or a derivative thereof, can be replaced with one or more of: polyethylene glycol (PEG), glycerin, glycerol, an oligomer or polymer of ethylene oxide, a methoxypolyethylene glycol, a polyethylene oxide, a polyoxyethylene, a simple polyol, 1,2-dihydroxypropane, 1,2-propanediol, methyl glycol, trimethyl glycol, or a derivative of any of the above.

One or more stabilizers can be used in the compositions disclosed herein. According to the claimed invention the stabilizer to be used is selected from propylene glycol, glycerol, an oligomer or polymer of ethylene oxide, a methoxypolyethylene glycol, 1,2-dihydroxypropane, methyl glycol or trimethyl glycol.

### pH

Each stock solution can be brought to an approximate pH of about 4.5 to about 9.5, about 5 to about 9, or about 3.5 to about 10.5, about 8.75 +/- 0.25, or about 8.25 +/- 0.25.

Stock solutions, CO+, CPD+, ProBio Foam, and CO+ wipes do not need to be brought to a certain pH. pH can be adjusted with, for example, glycolic acid, potassium hydroxide, sodium hydroxide, or any other solution known to one of skill in the art.

### TABLE 7: EXEMPLARY DILUTIONS OF STOCK SOLUTIONS

Each stock solution disclosed herein can be used with or without diluting the stock solution. Each stock can be diluted in an aqueous solution, for example, water. Any aqueous solution can be used as a diluent. The aqueous solution can be a mixture of two or more aqueous solutions.

Each stock solution can be diluted based on need (for example, how dirty the environment is) or application (for example, wipe, spray, mist, or mop). For example, if the surface needs a heavy cleaning the dilution will be less than a surface that needs a routine cleaning. In addition, any of the compositions disclosed herein can be diluted based on the species of the animal and/or the age of the animal that it is being used to treat. For example, W+ can be diluted to a greater extent in the water of a chicken, versus the water of a turkey, and W+ can be diluted more in an eight-week old broiler chicken than in a one-week old broiler chicken. One of skill in the art would be able to choose the appropriate dilution based on, for example, the animal, the age of the animal, the health of the animal, the nature of environmental challenges, the surface, the frequency of use, or type of application.

| **Composition Name** | **Dilute Stock Solutions to X** |
|---|---|
| | **Exemplary Dilutions if Needed** |
| A1+ | No dilution of Stock Solution. |
| | X=128 in **Table 2.** |
| | X=from about 50 to about 128 |
| | X=from about 2 to about 200 |
| A2+ | No dilution of Stock Solution. |
| | X=64 in **Table 2.** |
| | X=from about 32 to about 75 |
| | X=from about 10 to about 128 |
| B1+ | No dilution of Stock Solution. |
| | X=128 in **Table 2.** |
| | X=from about 50 to about 128 |
| | X=from about 2 to about 128 |
| CF+ | No dilution of Stock Solution. |
| | X=128 in **Table 2.** |
| | X=from about 50 to about 128 |
| | X=from about 2 to about 256 |
| C+ | No dilution of Stock Solution. |
| | X=30 in **Table 2.** |
| | X=from about 10 to about 40 |
| | X=from about 1 to about 128 |
| CO+ | No dilution of Stock Solution. |
| | X=30 in **Table 2.** |
| | X=from about 5 to about 40 |
| | X=from about 1 to about 128 |
| M+ | No dilution of Stock Solution. |
| | X=2 in **Table 2** |
| | X=from about 1 to about 2 |
| | X=from about 0 to about 50 |
| W+ | No dilution of Stock Solution. |
| | X=10,000 in **Table 2** |
| | X=from about 1,500 to about 20,000 |
| | X=from about 1 to about 50,000 |
| | X=from about 100 to about 100,000 |
| P+ | No dilution of Stock Solution. |
| | X=5,000 in **Table 2** |
| | X=from about 2,500 to about 20,000 |
| | X=from about 1,000 to about 50,000 |
| | X=from about 250 to about 100,000 |
| M+2 | No dilution of Stock Solution. |
| | X=4 in **Table 2** |
| | X=from about 2 to about 4 |
| | X=from about 0.1 to about 100 |
| M+2s | No dilution of Stock Solution. |
| | X=4 in **Table 2** |
| | X=from about 2 to about 4 |
| | X=from about 0.1 to about 100 |
| W+2 | No dilution of Stock Solution. |
| | X=20,000 in **Table 2** |
| | X=from about 3,000 to about 40,000 |
| | X=from about 1 to about 100,000 |
| W+2s | No dilution of Stock Solution. |
| | X=20,000 in **Table 2** |
| | X=from about 3,000 to about 40,000 |
| | X=from about 1. to about 100,000 |
| AC-C | No dilution of Stock Solution. |
| | X=30 in **Table 2** |
| | X=from about 5 to about 40 |
| | X=from about 2 to about 128 |
| AC-S | No dilution of Stock Solution. |
| | X=: 2 in **Table 2** |
| | X=from about 1 to about 2 |
| | X=from about 1 to about 50 |
| AC-L | X=64 in **Table 2** |
| | X=from about 32 to about 75 |
| | X=from about 5 to about 128 |
| CPD+ and CPD+ wipe | No dilution of Stock Solution. |
| D+t128 and | No dilution of Stock Solution. |
| D+t128 wipe | X=128 in **Table 2** |
| | X=from about 100 to about 150 |
| | X=from about 10 to about 256 |
| HC+ | No dilution of Stock Solution. |
| | X=1 in **Table 2** |
| | X=from about 1 to about 10 |
| | X=from about 0 to about 50 |
| D+h100 | No dilution of Stock Solution. |
| | X=100 in **Table 2** |
| | X=from about 75 to about 128 |
| | X=from about 10 to about 256 |
| Tingle | No dilution of Stock Solution. |
| | X=20 in **Table 2** |
| | X=from about 5 to about 20 |
| | X=from about 1 to about 100 |
| ProBio foam | No dilution of Stock Solution. |
| | X=10 in **Table 2** |
| | X=from about 2 to about 10 |
| | X=from about 1 to about 20 |
| A1+ wipe | No dilution of Stock Solution. |
| CO+ wipe | No dilution of Stock Solution. |
| J+ | No dilution of Stock Solution. |
| | X=from about 5 to about 200 |
| | X=from about 5 to about 20 |

### TREATMENT OR INTRODUCTION

Treatment or introduction of any one of the compounds of the disclosure can occur one time, more than one time, intermittently, continuously, or throughout the lifetime of the mammal or non-human mammal.

### WIPES

The products can be applied to a "wipe" or cloth that can be applied to a surface or an animal, or a part of an animal, for example, a teat of a dairy cow.

### SPRAYS, MISTS, AND AEROSOLS

All of the stock solutions (diluted or not diluted) can be applied as, for example, a spray, a mist, or as an aerosol. All of the stock solutions can be used in an aerosol form, or in a colloidal suspension of particles dispersed in air or gas.

### FOAMING PRODUCTS

All of the stock solutions (diluted or not diluted) that comprise one or more surfactants can be applied as a foam. Foam can be, for example, froth, spume, fizz, bubbles, head, lather, or suds.

### BIOFILM

Biofilm is a protective shelter created by pathogenic bacteria. Biofilms comprise, for example, microbial secretions, bacteria, viruses, dirt, corrosion products, and entrapped clay and soil materials. Biofilm include both beneficial and pathogenic bacteria. Examples of pathogenic species identified in biofilm include: Legionella, Listeria monocytogenes, Mycobacterium, E. coli, Enterococcus, Streptococcus, Salmonella, Staphylococcus, Cryptosporidium, Pseudomonas, Candida, and Nematode worms. Viruses and Fungi are also present in biofilm. Biofilm is one of the main causes of pathogen persistence. Biofilm growth is rapid - it doubles in approximately, 18-24 minutes (doubling every 21 minutes results in a colony of 5.9E+20).

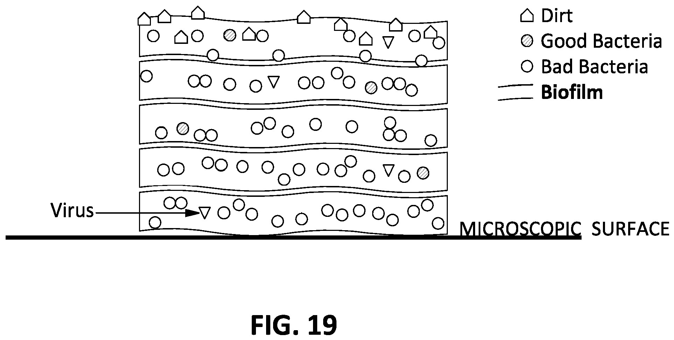

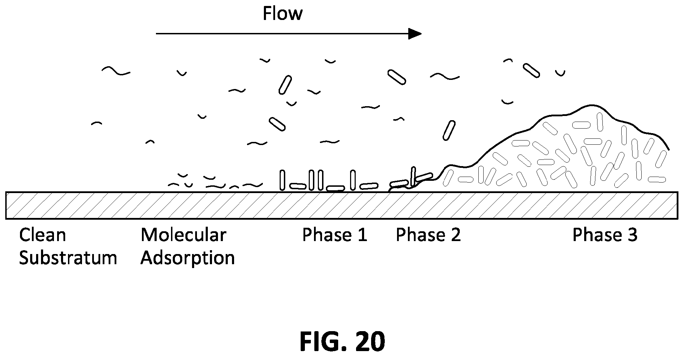

Biofilms are formed by microorganisms attaching and growing on a surface, see FIG. 19. This is the preferred method of growth for microorganisms. The biofilm provides the microorganisms with many advantages, for example, nutrients, community interactions, and protection from environmental stresses. The stages of formation of a biofilm, as shown in FIG. 20 are as follows: clean surface on immersion, conditioning film formation (molecular absorption), bacterial adhesion (phase 1), growth of and EPS production by the adhering bacteria (phase 2), and the mature biofilm (phase 3). (American Society for Microbiology, ASM News, 1992, 58:202-207).

Biofilms have been identified as the primary culprit in the biological contamination of drinking water distribution systems and industrial process water systems. Furthermore, biofilms are responsible for the loss of efficiency in heat exchangers and cooling towers and have also been identified as a persistent source of contamination and infection in medical implant devices and dental caries.

As discussed in Watnick P. and Kolter, R. (Biofilm, City of Microbes, Journal of Bacteriology (2000) p. 2675-2679), biofilms acquire transmissible, genetic elements at accelerated rates. There are many reports of accelerated rates of conjugation in bacterial biofilms. This suggests that evolution by horizontal transfer of genetic material may occur rapidly in a biofilm, making it the perfect milieu for emergence of new pathogens by acquisition of antibiotic resistance, virulence factors, and environmental survival capabilities.

Biofilm-associated cells are more resistant to many toxic substances such as antibiotics, sanitizers, disinfectants, cleaners, and detergents. There is evidence that decreased diffusion into the biofilm, decreased bacterial growth rate in a biofilm, biofilm-specific substances such as exopolysaccharide, and the quorum-sensing specific effects may be reasons for this resistance.

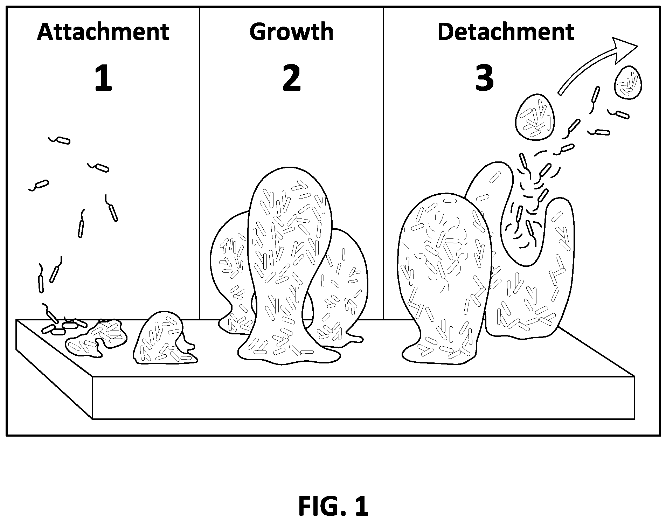

FIG. 1 illustrates three steps in biofilm formation on an exemplary flat surface (Center for Biofilm Engineering at MSU-Bozeman, P. Dirckz, (2003)).

A floating pathogen cell (for example, Staphylococcus aureus, enterobacteriaceae (Salmonella), or E. coli) is said to be planktonic, or free floating. The first thing that a pathogen wants to do is to attach (Attachment) to a surface and begin the process of creating a colony through redoubling (Growth) approximately every 21 minutes. In order to anchor to the surface, the pathogen will secrete an extracellular polymeric substance that is a sticky substance that also provides a protective home for the colony, which can also comprise, for example, viruses and fungi. The colony will grow to the point where it will begin to shed (Detachment) both individual pathogens and small sub-colonies that will then proceed to establish their own colonies. Biofilm formed by this mechanism occurs, for example, within water lines, hard surfaces, soft surfaces, virtually anywhere that a pathogen cell can land and begin the attachment and growth process.

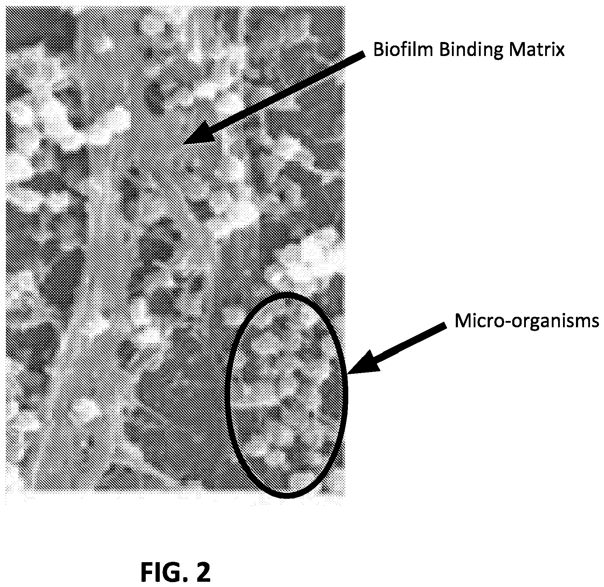

Biofilms create a "shelter" for microorganisms, such as bacteria, archae, fungi, viruses and algae, to live in. Shown in FIG. 2 is a biofilm binding matrix. Examples of biofilm binding matrixes are extracellular polymeric substances (EPS), polysaccharides, exopolysaccharides, glycoproteins, glycolipids, and proteins. Also, shown in FIG. 2 are micro-organisms, for example, bacteria, archaea, fungi, protozoa, and algae.

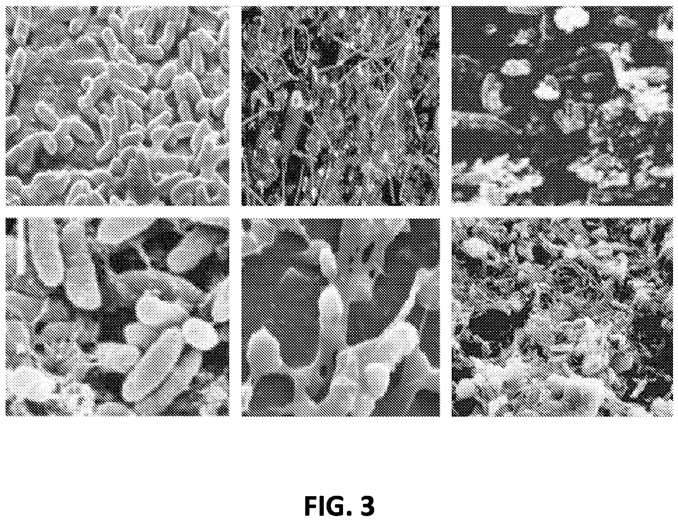

Biofilm is ubiquitous, it is found virtually everywhere. Biofilm can form in numerous locations and on numerous surfaces. Biofilm is present in our waterways, food processing methods, water distribution systems, body (e.g. lungs and teeth), and even on our contact lenses. Biofilm can form on any surface, for example, plastic, stainless steel, metal, wood, ceramic, glass, paint, skin, feathers - virtually anywhere there are planktonic pathogens present they can start a biofilm colony. FIG. 3 shows images from a scanning electron microscope of biofilm found at different locations. The top panel shows, left to right, biofilm found in food processing, waterways, and water distribution systems. The bottom panel shows, left to right, biofilm found in human lungs, human and animal teeth, and contact lenses. These examples are a minute fraction of all of the locations and surfaces that biofilm forms and resides.

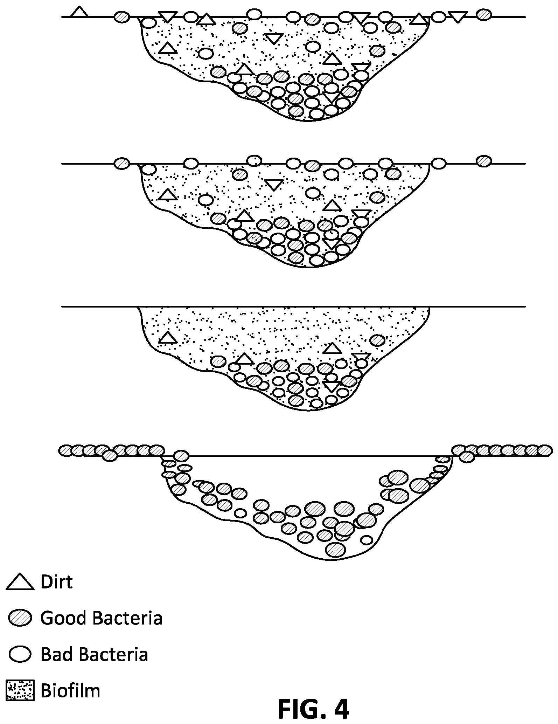

As shown in FIG. 4, the first panel (starting at the top of the figure), before cleaning of a surface, biofilm comprises, inter alia, dirt, and beneficial bacteria (good bacteria) and pathogenic bacteria (bad bacteria). The second panel of FIG. 4 shows how detergent (for example, Formula 409, Clorox Green Works, Ajax, or any known detergent) removes surface dirt, but has little or no effect on disrupting the biofilm or reducing the number of bacteria. When an enzyme (for example, an amylase, a protease, or a lipase) is added in combination with a detergent, see the third panel, surface dirt and bacteria are partially removed, with little effect on breaking down the biofilm and reducing the number of encapsulated bacteria. However, when a composition of the disclosure is used, or added, surface dirt, bacteria, and biofilm are greatly reduced, and the composition reduces and/or prevents the recolonization of pathogens on the surface, see the last panel (or bottom panel) of FIG. 4. FIG. 4 teaches how current protocols for cleaning a surface (detergent plus enzyme) are not effective in removing biofilm.

FIG. 4 is a schematic showing the effectiveness of the compositions described herein. For example, as shown in the last panel (or bottom panel), when composition C+ is added, surfactants remove the surface starches, sugars, and fats to allow access by enzymes (a protease and a beta amylase) and a mix of Bacillus species to the biofilm surface. For example, composition C+ is applied to a surface in a liquid form. While the surface is wet the enzymes are an effective means of beginning the biofilm dissolution and removal process. Once the enzymes dry, since they are proteins, their effectiveness is stopped. The probiotic bacteria are able to work in wet and dry conditions and have the ability to continue to dissolve biofilm for a long period of time. For example, the probiotic bacteria can continue to dissolve biofilm as long as they have nutrients to consume. If a nutrient source (biofilm) disappears, the probiotic bacteria can still prevent recolonization of biofilm on the surface for several days.

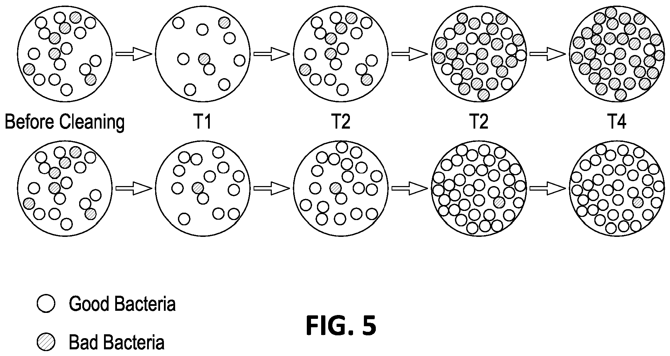

FIG. 5 is a schematic of what happens to beneficial bacteria (good bacteria) and pathogenic bacterial (bad bacteria) after cleaning with a sanitizer (e.g. disinfectant) (top panel), versus a composition of the disclosure (bottom panel). The good bacteria are represented by light circles. The pathogenic bacteria are represented by dark circles. The petri dishes in both the top panel and bottom panel begin ("before cleaning") with an identical composition of bacteria at similar concentrations. Cleaning with the sanitizer will kill the majority of the bacteria (both good and bad) residing on the surface (T1), but a residual number of bacteria will remain. Sanitizers kill most bacteria and leave behind protein remains that serve as nutrients for the "bad" bacteria. The pathogenic bacteria take advantage of the food source and out compete the surviving beneficial bacteria, thereby increasing the number of pathogenic bacteria in the biofilm colony and enabling the pathogenic bacteria to rapidly grow the colony (for example, see top panel T2 to T4).

In stark contrast, the surfactant present in the composition of the disclosure kills only a small percentage of the surface bacteria, leaving behind a probiotic layer that works to consume any protein (food) and the biofilm present, and simultaneously covers the surface contacted with the probiotics of the composition, thus preventing planktonic pathogens from being able to attach and form a colony. FIG. 5 illustrates of how over a period of time (before cleaning of the surface to approximately 24 hours (T4) after cleaning of the surface) how the number of bad bacteria increases in the top panel (T2 to T4), whereas the number of good bacteria increases in the bottom panel (T2 to T4). Examples of commonly used disinfectants are bleach, oxidizers, quaternary ammonium, chlorhexidine, glutaraldehyde, phenols, pine-oil derivatives, and others known to one skilled in the art.

### SURFACTANTS

Surfactants are, for example, compounds that lower the surface tension (or interfacial tension) between two liquids or between a liquid and a solid. Surfactants may act, for example, as soaps, detergents, wetting agents, emulsifiers, foaming agents, and dispersants.

### PROBIOTIC BACTERIA

Several different strains of Bacillus can be used in the compositions described herein, several of which are described below. In addition, other probiotic strains such as Enterococcus can be used in the compositions described herein. One of skill in the art would be able to select, test, and determine whether a strain or species of Bacillus was effective in reducing a pathogen from an environment or reducing a biofilm from an environment.

### BACILLUS

Bacillus is a genus of Gram-positive, rod-shaped (bacillus) bacteria and a member of the phylum Firmicutes. Bacillus species can be obligate aerobes (oxygen reliant), or facultative anaerobes (having the ability to be aerobic or anaerobic). They will test positive for the enzyme catalase when there has been oxygen used or present. Ubiquitous in nature, Bacillus includes both free-living (nonparasitic) and parasitic pathogenic species. Under stressful environmental conditions, the bacteria can produce oval endospores that are not true "spores", but to which the bacteria can reduce themselves and remain in a dormant state for very long periods. These characteristics originally defined the genus, but not all such species are closely related, and many have been moved to other genera of the Firmicutes.

Many strains of Bacillus are categorized as GRAS. "GRAS" is an acronym for the phrase Generally Recognized As Safe. Under sections 201(s) and 409 of the Federal Food, Drug, and Cosmetic Act (the Act), any substance that is intentionally added to food is a food additive, that is subject to premarket review and approval by the Food and Drug Administration (FDA), unless the substance is generally recognized, among qualified experts, as having been adequately shown to be safe under the conditions of its intended use, or unless the use of the substance is otherwise excluded from the definition of a food additive. Under sections 201(s) and 409 of the Act, and FDA's implementing regulations in 21 CFR 170.3 and 21 CFR 170.30, the use of a food substance may be GRAS either through scientific procedures or, for a substance used in food before 1958, through experience based on common use in food.

According to the invention, the Bacillus species used are selected from Bacillus subtilis, Bacillus licheniformis, Bacillus pumilus, Bacillus coagulans, Bacillus amyloliquefaciens, Bacillus megaterium, or Bacillus mojavensis.

### BACILLUS SUBTILIS (B. SUBTILIS)

B. subtilis is categorized as GRAS. B. subtilis, as with many in the Bacillus genus, is an extremely common bacterium. It is found in soil, water, air, and decomposing plant matter. Bacteria in the Bacillus genus are spore-forming, which means that they create a thick wall that surrounds their DNA and other internal cell structures. In this way, they are very hardy and impervious to extreme temperatures, chemicals, environmental factors, and even some types of radiation. This makes them excellent for use in industrial processes.

B. subtilis is a Gram-positive, catalase-positive bacterium, found in soil and the gastrointestinal tract (GIT) of most animal, ruminants and humans. B. subtilis is rod-shaped, and can form a tough, protective endospore, allowing it to tolerate extreme environmental conditions. B. subtilis has historically been classified as an obligate aerobe, though evidence exists that it is a facultative aerobe.

The concentration of B. subtilis added to the Product Stock Solutions of Table 2, can vary. For example, 10 billion CFU/mg to 500 billion CFU/mg of B. subtilis can be added to the Product Stock Solutions of Table 2.

### BACILLUS LICHENIFORMIS (B. LICHENIFORMIS)

B. licheniformis is categorized as GRAS. B. licheniformis is a Grain-positive and rod-shaped bacterium. It is a motile bacterium, a facultative anaerobe (unlike other bacilli that are typically aerobic), and closely related to Bacillus subtilis. B. licheniformis produces endospores, and is usually found in the soil as spores. It is a microorganism that is usually associated with plants and plant materials. It is found on bird feathers, especially chest and back plumage, and most often in ground-dwelling birds. It is a mesophilic bacterium. Its optimal growth temperature is around 30°C, though it can survive at much higher and much lower temperatures. The optimal temperature for enzyme secretion is 37°C. It can exist in spore form to resist harsh environments, or in a vegetative state when conditions are good.

The concentration of B. licheniformis added to the Product Stock Solutions of Table 2, can vary. For example, 10 billion CFU/mg to 500 billion CFU/mg of B. licheniformis can be added to the Product Stock Solutions of Table 2.

### BACILLUS PUMILUS (B. PUMILUS)

B. Pumilus is categorized as GRAS. B. Pumilus is a Gram-positive, aerobic, spore-forming bacillus commonly found in soil. B. pumilus spores generally show high resistance to environmental stresses, including UV light exposure, desiccation, and the presence of oxidizers such as hydrogen peroxide.

The concentration of B. Pumilus added to the Product Stock Solutions of Table 2, can vary. For example, 10 billion CFU/mg to 500 billion CFU/mg of B. Pumilus can be added to the Product Stock Solutions of Table 2.

### B. COAGULANS

B. coagulans is categorized as GRAS. B. coagulans is a Gram-positive rod (0.9 by 3.0 to 5.0 µm in size), catalase positive, spore-forming, motile, and a facultative anaerobe. It may appear Gram-negative when entering the stationary phase of growth. Bacillus coagulans produces lactic acid and, as a result, is often misclassified as lactic acid bacteria such as lactobacillus. Unlike lactic acid bacteria such as lactobacillus or bifidobacteria, Bacillus coagulans forms reproductive structures called spores. Spores are actually an important factor in telling Bacillus coagulans apart from lactic acid bacteria.

The concentration of B. coagulans added to the Product Stock Solutions of Table 2, can vary. For example, 10 billion CFU/mg to 500 billion CFU/mg of B. coagulans can be added to the Product Stock Solutions of Table 2.

In addition to the mix of Bacillus strains in the disclosed compositions, the following bacterial strains or species can be added to any of the novel compositions described herein, One or more additional bacterial strains or species can be added to the mix of Bacillus strains while making the mix of Bacillus strains, or added to a final stock solution (for example, C+) after the mix of Bacillus strains has been added to the final stock solution, In addition, one or more of the following bacterial strains or species can be added prior to or after dilution of a stock solution.

Exemplary bacterial strains or species that can be added to the compositions disclosed herein are, Bacillus coagulans, Bacillus subtilis, Bacillus licheniformis, Bacillus pumilis, Bacillus amyloliquefacicns, Bacillus megaterium, Bacillus mojavensis, Lactobacillus casei, Lactobacillus reuterii, Bifidobacterium bifidum, or Streptococcus thermophiles.

### ADDITIONAL PROBIOTIC BACTERIAL STRAINS

An exemplary list of probiotic bacteria that can be used in or added to the compositions disclosed herein, are listed below. These probiotic bacteria are GRAS, as recognized by the FDA.

A probiotic bacteria, useful in the compositions of the disclosure can be selected from any one or more of the following genera: Aspergillus, Bacillus, Bacteroides, Lactobacillus, Bifidobacterium, Leuconostoc, Pediococcus, Saccharomyces, or Streptococcus.

A probiotic bacteria, useful in the compositions of the disclosure can be selected from any one or more of the following species: Aspergillus niger, Aspergillus oryzae, Bacillus coagulans, Bacillus lentus, Bacillus lincheniformis, Bacillus pumilus, Bacillus subtilis, Bacteroides amylophilus, Bacteroides capillosus, Bacteroides ruminocola, Lactobacillus cellobiosus, Lactobacillus curvatus, Lactobacillus delbruekii, Lactobacillus fermentum, Lactobacillus lactis, Lactobacillus plantarum, Bacteroides suis, Bifidobacterium adolescentis, Bifidobacterium animalis, Bifidobacterium bifidum, Bifidobacterium infantis, Bifidobacterium longum, Bifidobacterium theniiophilum, Lactobacillus acidophilus, Lactobacillus brevis, Lactobacillus bulgaricus, Lactobacillus casei, Lactobacillus reuterii, Leuconostoc mesenteroides, Pediococcus acidilacticii, Pediococcus cerevisiae (damnosus), Pediococcus pentosaceus, Propionibacterium freudenreichii, Propionbacterium sherman.ii, Saccharomyces cerevisiae, Streptococcus cremoris, Streptococcus diacetylactis, Streptococcus faecium, Streptococcus intermedius, Streptococcus lactis, or Streptococcus thermophilus.

### AMYLASES

Beta amylase is used in the compositions disclosed herein. However, one or more amylases can be used in the compositions disclosed herein. Any amylase known to one of skill in the art, such as those disclosed herein, can be used in the compositions of the disclosure. Exemplary amylases are α-amylases. β-Amylase, and γ-Amylase.

An amylase is any member of a class of enzymes that catalyze the hydrolysis (splitting of a compound by addition of a water molecule) of starch into smaller carbohydrate molecules such as maltose (a molecule composed of two glucose molecules). Two categories of amylases, denoted alpha and beta, differ in the way they attack the bonds of the starch molecules.

Alpha-amylase is widespread among living organisms. In the digestive systems of humans and many other mammals, an alpha-amylase called ptyalin is produced by the salivary glands, whereas pancreatic amylase is secreted by the pancreas into the small intestine.

Ptyalin is mixed with food in the mouth, where it acts upon starches. Although the food remains in the mouth for only a short time, the action of ptyalin continues for up to several hours in the stomach until the food is mixed with the stomach secretions, the high acidity of which inactivates ptyalin. Ptyalin's digestive action depends upon how much acid is in the stomach, how rapidly the stomach contents empty, and how thoroughly the food has mixed with the acid. Under optimal conditions as much as 30 to 40 percent of ingested starches can be broken down to maltose by ptyalin during digestion in the stomach.

When food passes to the small intestine, the remainder of the starch molecules are catalyzed mainly to maltose by pancreatic amylase. This step in starch digestion occurs in the first section of the small intestine (the duodenum), the region into which the pancreatic juices empty. The by-products of amylase hydrolysis are ultimately broken down by other enzymes into molecules of glucose, which are rapidly absorbed through the intestinal wall.

Beta-amylases are present in yeasts, molds, bacteria, and plants, particularly in the seeds. They are the principal components of a mixture called diastase that is used in the removal of starchy sizing agents from textiles and in the conversion of cereal grains to fermentable sugars.

### PROTEOLYTIC ENZYMES

Alkaline protease is used in the compositions disclosed herein. However, one or more proteases can be used in the compositions disclosed herein. Any protease known to one of skill in the art, such as those disclosed herein, can be used in the compositions of the disclosure. Exemplary proteases are serine proteases, cysteine proteases, aspartate proteases, threonine proteases, glutamic acid proteases, metalloproteases, or asparagine peptide lyases.

Proteolytic enzymes, also called proteases, proteinases, or peptidases, are any of a group of enzymes that break the long chainlike molecules of proteins into shorter fragments (peptides) and eventually into their components, amino acids. Proteolytic enzymes are present in bacteria, archaea, certain types of algae, some viruses, and plants; they are most abundant, however, in animals.

There are different types of proteolytic enzymes, which are classified according to sites at which they catalyze the cleavage of proteins. The two major groups are the exopeptidases, which target the terminal ends of proteins, and the endopeptidases, which target sites within proteins. Endopeptidases employ various catalytic mechanisms; within this group are the aspartic endopeptidases, cysteine endopeptidases, glutamic endopeptidases, metalloendopeptidases, serine endopeptidases, and threonine endopeptidases. The term oligopeptidase is reserved for those enzymes that act specifically on peptides.

Among the best-known proteolytic enzymes are those that reside in the digestive tract. In the stomach, protein materials are attacked initially by a gastric endopeptidase known as pepsin. When the protein material is passed to the small intestine, proteins, which are only partially digested in the stomach, are further attacked by proteolytic enzymes secreted by the pancreas. These enzymes are liberated in the small intestine from inactive precursors produced by the acinar cells in the pancreas. The precursors are called trypsinogen, chymotrypsinogen, proelastase, and procarboxypeptidase. Trypsinogen is transformed to an endopeptidase called trypsin by an enzyme (enterokinase) secreted from the walls of the small intestine. Trypsin then activates the precursors of chymotrypsin, elastase, and carboxypeptidase. When the pancreatic enzymes become activated in the intestine, they convert proteins into free amino acids, which are easily absorbed by the cells of the intestinal wall. The pancreas also produces a protein that inhibits trypsin. It is thought that in this manner the pancreas protects itself from autodigestion.

### LIPASES

A lipase can be is used in the compositions disclosed herein. However, one or more lipases can be used in the compositions disclosed herein. Any lipase known to one of skill in the art, such as those disclosed herein, can be used in the compositions of the disclosure.

Lipases are any of a group of fat-splitting enzymes found in the blood, gastric juices, pancreatic secretions, intestinal juices, and adipose tissues. Lipases hydrolyze triglycerides (fats) into their component fatty acid and glycerol molecules.

Initial lipase digestion occurs in the lumen (interior) of the small intestine. Bile salts reduce the surface tension of the fat droplets so that the lipases can attack the triglyceride molecules. The fatty acid and glycerol molecules are then taken up into the epithelial cells that line the intestinal wall, where they are resynthesized into triglycerides for transport to muscles and adipose tissues.

Exemplary lipases are bile salt-dependent lipase (bsdl), pancreatic lipase (PNLIP), lysosomal lipase (LIPA), hepatic lipase (LIPC), lipoprotein lipase (LPL or "LIPD"), hormone-sensitive lipase (LIPE), gastric lipase (LIPF), endothelial lipase (LIPG), pancreatic lipase related protein 2 (PNLIPRP2 or "PLRP2"), or pancreatic lipase related protein 1 (PNLIPRP1 or "PLRP1").

### ADDITIONAL ENZYMES OR COMPOUNDS

Additional Enzymes that can be added to any of the compositions described herein are any enzyme that is capable of breaking down sugars, starches, or fats. For example, a lipase or a xylanase. A lipase can be added to any of the stock solutions at a concentration of from about 0.5% to 5.0% (final volume), or from about 0.25% to about 10% (final volume). Any enzyme or compound (chemical) useful in breaking down or digesting human or animal food products can be used in the compositions described herein. For example, the following enzymes or compounds can be used, betaine HCL that activates the protein digesting enzyme pepsin in the stomach, cellulose that breaks down cellulose, or alpha-galactosidase that facilitates the digestion of seeds, soy, and beans. The additional enzyme or compound can be added to the stock solution of a composition (TABLE 2) or to a diluted version of the stock solution (TABLE 7).

### PATHOGENS

A pathogen is anything that causes a disease. Pathogens include, for example, bacteria, viruses, or fungi. A pathogen can be, for example, a pathogenic bacteria or a virus.

A pathogen can be, for example, bovine herpesvirus 1, 2 (infections rhinotracheitis), bovine viral diarrhoea virus (pestivirus), parainfluenza virus (PT 3), bovine respiratory syncytial virus, Mannheimia haemolytica, Pasteurella multocida, Histophilus somnus, actinomyces, actinobacillus, mycoplasma, Dichelobacter nodosus, or Fusobacterium necrophorum.

### PATHOGENIC BACTERIA

Described below are numerous exemplary pathogenic bacteria whose numbers or population can be decreased or eliminated by the compositions disclosed herein. This list is non-exhaustive. One skilled in the art could identify the presence of any pathogenic bacteria that is not listed herein, or not yet evolved, and determine whether the compounds of the disclosure are effective against it. New resistant strains evolve every day.

Pathogen resistance to antibiotics continues to increase and new resistant strains are also increasing. Pathogenic Bacteria can include multidrug-resistant organisms (MDROs).

Pathogenic bacteria can be for example, Pseudomonas aeruginosa, Clostridium difficile (C. Diff), Neisseria gonorrhaeae, Carbapenem-resistant Enterobacteriaceae (CRE), Drug-resistant Streptococcus pneumoniae, Drug-resistant Campylobacateria, Drug-resistant non-typhoidal Salmonella, Methicillin-resistant Staphylococcus aureus (MRSA), Drug-resistant Shigella, Extended-spectrum Enterobacteriaceae (ESBL), Vancomycin-resistant Enterococcus (VRE), Multidrug-resistant Acinetobacteria, Multidrug-resistant Pseudomonas aeruginosa, Drug-resistant Salmonella serotype Typhi, Fluconazole-resistant Candida, Drug-resistant Tuberculosis, Clindamycin-resistant Group B Streptococcus, Erythromycin-resistant Group A. Streptococcus, or Vancomycin-resistant Staphylococcus aureus.

Pneumococcal disease, whether or not it is resistant to antibiotics, is a major public health threat. Pneumococcal disease causes four million disease patient cases and 22,000 deaths annually. Some Staphylococcus strains are resistant to vancomycin, leaving few or no other treatment options.

### Extended-Spectrum Beta-lactamase (ESBL) Producing Bacteria

ESBLs are enzymes that mediate resistance to extended-spectrum (third generation) cephalosporins (e.g., ceftazidime, cefotaxime, and ceftriaxone) and monobactams (e.g., aztreonam).

### SALMONELLA

Salmonella bacteria are a genus of rod-shaped bacteria of the Enterobacteriaceae family. Two exemplary species of Salmonella are Salmonella bongori and Salmonella enterica.

Salmonella serotype Enteritidis (SE) is one of the most common serotypes of Salmonella bacteria reported worldwide. Eggs have been the most common food source linked to SE infections. SE can be inside perfectly normal-appearing eggs. If eggs contaminated with SE are eaten raw or lightly cooked (runny egg whites or yolks), the bacterium can cause illness. Since the early 2000s, poultry has also been found to be a common food source for SE infections. Other identified sources linked to SE infections include raw milk, pork, beef, sprouts, and raw almonds.

Salmonella bongori is primarily associated with cold-blooded animals and infrequently colonize the intestines of warm-blooded animals.

### STAPHYLOCOCUS AUREUS

Staphylococcus aureus is a gram-positive coccal bacterium that is a member of the Finnicutes phylum, and is frequently found in the respiratory tract and on the skin of a mammal.

Staphylococcus aureus is a type of bacteria that about 30% of people carry in their noses. Most of the time, staph does not cause any harm; however, sometimes Staphylococcus aureus causes infections. In healthcare settings, these infections can be serious or fatal, including: bacteremia or sepsis when bacteria spread to the bloodstream; pneumonia, which predominantly affects people with underlying lung disease including those on mechanical ventilators; endocarditis (infection of the heart valves), which can lead to heart failure or stroke; or osteomyelitis (bone infection), which can be caused by Staphylococcus aureus traveling in the bloodstream or put there by direct contact such as following trauma (puncture wound of foot or intravenous (IV) drug abuse).

Staphylococcus aureus can also become resistant to certain antibiotics. These drug-resistant Staphylococcus aureus infections include: Methicillin-resistant Staphylococcus aureus (MRSA), Vancomycin-intermediate Staphylococcus aureus (VISA), and Vancomycin-resistant Staphylococcus aureus (VRSA)

### COLIFORM

Coliforms are a group of bacteria found in plant material, water, and soil. Coliforms are also present in the digestive tracts and feces of humans and animals. Some rare types of coliforms, such as E. coli O157:117, can cause serious illness.

### MRSA (METHICILLIN-RESISTANT STAPHYLOCOCCUS AUREUS)

Methicillin-resistant Staphylococcus aureus (MRSA) is a type of staphylococcus or "staph" bacterium that is resistant to many antibiotics. Staph bacteria, like other kinds of bacteria, normally live on your skin and in your nose, usually without causing problems. For example, MRSA infections can result in skin infections. But if these bacteria become resistant to antibiotics, they can cause serious infections, especially in people who are ill or weak. In medical facilities, MRSA causes life-threatening bloodstream infections, pneumonia and surgical site infections. MRSA is different from other types of staph because it cannot be treated with certain antibiotics such as methicillin.

MRSA infections are more difficult to treat than ordinary staph infections. This is because the strains of staph known as MRSA do not respond well to many common antibiotics used to kill bacteria. When methicillin and other antibiotics do not kill all of the bacteria causing an infection, the surviving bacteria are resistant to the antibiotic and it becomes necessary to try a different antibiotic to get rid of the infection.

### VRE (VANCOMYCIN-RESISTANT ENTEROCOCCUS)

Vancomycin-resistant enterococci (VRE) are a type of bacteria called enterococci that have developed resistance to many antibiotics, especially vancomycin. Enterococci bacteria live in the intestines and on the skin of a mammal, often without causing problems. But if they become resistant to antibiotics, they can cause serious infections, especially in mammals that are ill or weak. These infections can occur anywhere in the body. Some common sites include the intestines, the urinary tract, and wounds.

### VISA (VANCOMYCIN-INTERMEDIATE-STAPHYLOCOCCUS AUREUS)

Vancomycin-intermediate Staphylococcus aureus (also called VISA) and Vancomycin-resistant Staphylococcus aureus (also called VRSA) are specific types of antimicrobial-resistant bacteria. Persons who develop this type of staph infection may have underlying health conditions (such as diabetes and kidney disease), tubes going into their bodies (such as cadieters), previous infections with methicillin-resistant Staphylococcus aureus (MRSA), and recent exposure to vancomycin and other antimicrobial agents.

### CRE (CARBAPENEM-RESISTANT ENTEROBACTERIACEAE)

CRE, which stands for carbapenem-resistant Enterobacteriaceae, are a family of bacteria that are difficult to treat because they have high levels of resistance to antibiotics. CRE kills 50% of all patients who are infected. Klebsiella species and Escherichia coli (E. coli) are two examples of Enterobacteriaceae, a normal part of the human gut bacteria, which can become carbapenem-resistant. Types of CRE are sometimes known as KPC (Klebsiella pneumoniae carbapenemase) and NDM (New Delhi Metallo-beta-lactamase). KPC and NDM are enzymes that break down carbapenems and make them ineffective.

CRE have become resistant to nearly all the available antibiotics. Almost half of all hospital patients who get bloodstream infections from CRE bacteria die from the infection.

### LISTERIA MONOCYTOGENES (L. MONOCYTOGENES)

Listeria monocytogenes is the bacterium that causes the infection listeriosis. It is a facultative anaerobic bacterium, capable of surviving in the presence or absence of oxygen. Listeria is a gram-positive rod-shaped bacterium that can grow under either anaerobic (without oxygen) or aerobic (with oxygen) conditions. Of the six species of Listeria, only L. monocytogenes causes disease in humans. These bacteria multiply best at 86-98.6 degrees F (30-37 degrees C), but also multiply better than all other bacteria at refrigerator temperatures, something that allows temperature to be used as a means of differentiating Listeria from other contaminating bacteria.

L. monocytogenes is found in soil and water. Listeriosis is food poisoning caused by eating foods contaminated with the L. monocytogenes bacterium. In pregnant women, the infection can result in miscarriage, premature delivery, and serious infection of the newborn, or even stillbirth. Listeriosis affects mainly pregnant women, newborns, the elderly, and adults with impaired immune systems.

Called an "opportunistic pathogen," Listeria is noted to cause an estimated 2,600 cases per year of severe invasive illness. Foodborne illness caused by Listeria monocytogenes has raised significant public health concern in the United States, Europe, and other areas of the world.

### BOVINE RESPIRATORY DISEASE (BRD)

Bovine Respiratory Disease (BRD) is the most common and costly disease affecting the North American beef cattle industry. In the broadest sense, BRD refers to any disease of the upper or lower respiratory tracts. BRD is commonly associated with infections of the lungs causing pneumonia in recently weaned and feedlot cattle, nursing beef calves, housed dairy calves, and lactating dairy cows. BRD can account for 65-80% of the morbidity and 45-75% of the mortality in some feedlots.

BRD is a complex multi-factorial disease that involves an interaction between several factors, including host factors (characteristics of an animal that make it more prone to the disease), environmental factors (for example, transport, commingling, temperature fluctuations, crowding, ventilation, auction-sourced), and infectious agents (for example, pathogens, bacteria, parasites, and viruses).

### LAMINITIS

Laminitis is a disease that affects the feet of hooved animals (ungulates) and it is found mostly in horses and cattle. Clinical signs can include, for example, hairy wart, foot root, foot tenderness progressing to inability to walk, increased digital pulses, and increased temperature in the hooves. Severe cases with outwardly visible clinical signs are known by the colloquial term founder, and progression of the disease may lead to perforation of the coffin bone through the sole of the hoof, requiring aggressive treatment or euthanasia.

### VIRUSES

A virus is a type of pathogen. Viruses can also live in biofilm along with pathogenic bacteria. Described below are numerous exemplary viruses whose numbers or population can be decreased or eliminated by the compositions disclosed herein. This list is non-exhaustive. One skilled in the art could identify the presence of any virus that is not listed herein, or not yet evolved, and determine whether the compounds of the disclosure are effective against it. Exemplary viruses include MRSA, porcine enteritis virus, viruses that infect mammals and humans, porcine viruses, avian viruses (for example, avian influenza), bovine viruses, hemorrhagic enteritis virus, porcine respiratory virus, or coronavirus. Other exemplary viruses include, bovine herpesvirsus (IBR); bovine parainfluenza virus (PI-3); bovine respiratory syncytial virus (BRSV); bovine viral diarrhea virus (BVD), and bovine coronavirus (BCV).

### APPLICATION OF COMPOSITIONS

The novel compositions disclosed herein can be applied in several different ways. For example, the compositions can be sprayed, misted, hosed, foamed, fogged, electrostatically applied or poured onto a surface. As mentioned above, the compositions can also be applied using a wipe or a cloth. The compositions disclosed herein can applied by using a water line, water drip, valve, nozzle, tank, nipple drinker, bell drinker, trough, doser, or automated proportioning system. One of skill in the art would know other ways to apply or use the compositions disclosed herein, and could easily determine which type would be appropriate for any of the disclosed compositions. The novel compositions disclosed herein can be added to the water supply of any livestock or mamma A use in the treatment of humans or animals is however not part of the claimed invention.

Treating the environment, including any surface, space, or air space, can include, for example, any method of applying the compositions as discussed herein. A treating step or introducing step, as disclosed herein can be repeated two or more times. A treating step or introducing step can be repeated as many times and for as long a period as deemed necessary by one skilled in the art to treat the non-human mammal or mammal. The treatment of humans or animals is however not part of the claimed invention.

### ENVIRONMENT

The environment of a human or non-human mammal can be, for example, any surface, space, air space, or water supply that is in contact with or in close proximity to the mammal or non-human mammal. The environment of a human can be, for example, a work place, a home, or any location that a human can be present in. The environment of a non-human mammal can be, for example, any surface, air, or air space found in a farm, a farm enclosure, a hatchery, a processing plant, or a storage facility. The environment of a non-human mammal can be, for example, any place that a non-human mammal comes in contact with or can be present in. The environment of a fish can be, for example, any surface, space, air space, or water supply that is in contact with or in close proximity to the fish.

### SURFACE, SPACE, OR AIR SPACE

Any surface, space, or air space, present in a private or public space can be cleaned by the compositions of the disclosure. Any surface, space, or air space, involved in taking care of any mammal (including a human), can be cleaned by the compositions of the disclosure. For example, the compositions disclosed herein can be applied to any surface of a processing plant, a farm, a school, a home, a commercial building, a factory, a store, a clinic or a hospital. A surface can be, for example, round, rectangular, triangular, oval, uneven, flat, vertical, or horizontal. A surface can be made of any material. For example, metal, plastic, wood, ceramic, glass, stainless steel, steel, cast iron, plaster, paint, other various metals, or cement. A surface can be made of a hard material or a soft material, or made of a combination of a hard material and a soft material.

In regards to livestock in particular, the surface, space, or air space can be located in or around, for example, a bam, a nest, an egg, a boot wash tray, a water source, transportation equipment, any equipment that comes into contact with the livestock, a fogging room, an incubator, a setting tray, or a water line. In addition, the surface, space, or air space can be any surface, space, or air space that is used during processing, transportation, or delivery of the carcass.

The surface, space, or air space can be present in or around, for example, a pipe, a water line, a medical implant, a dental or medical device, equipment, kitchen, bathroom, tubing, heat exchanger, cooling tower, water distribution system, drinking water, hazardous waste sites, or municipal and industrial water and wastewater systems.

### APPLICABLE INDUSTRIES - LIVESTOCK

The novel compositions disclosed herein can be used to improve the overall health of any mammal, for example, livestock. Exemplary livestock include: poultry, pigs, piglets, poults, turkeys, sheep, goats, dairy cattle, beef cattle, ruminants, chickens, broiler chickens, hens, geese, ducks, any swine species, any porcine species, any bovine species, koi, shrimp, salmon, other farmed fish, or any avian species.

All stages of the life cycle of the livestock described herein can be improved by the compositions disclosed herein. For example, from piglet to pig, egg to chicken, egg to turkey, call to cow, calf to steer, breeding to broiler chicken, breeding to turkey, breeding to pig, insemination to horse, insemination to cow, breeding to duck, breeding to sheep, breeding to goat, and breeding to steer. Such methods and uses in the treatment of humans and or animals are however not part of the claimed invention.

### APPLICABLE INDUSTRIES - HOME AND COMMERCIAL

The novel compositions disclosed herein can be used to improve the overall health of humans in any public or private space. The compositions of the disclosure can be used, for example, to reduce, eliminate, or prevent biofilm growth on any surface, space, or air space in public buildings, offices, private homes, businesses, and all modes of public transportation. By reducing, eliminating, or preventing biofilm growth, on any surface, space, or air space, the spread of contagious and often deadly diseases can be reduced or prevented. Restaurants will have cleaner surfaces in contact with humans (for example, menus, tables, and door handles) and food preparation facilities. The surfaces of buses and trains (for example, doors and handles) will not be incubators for the spread of disease, and playgrounds and schools will not be incubators for the spread of disease amongst children and their families.

### APPLICABLE INDUSTRIES - MEDICAL AND HUMAN HEALTH CARE

Biofilms are a huge problem in the health care industry. Biofilm is present on almost every surface, dry or moist. The compositions of the disclosure can be used, for example, to reduce, eliminate, or prevent biofilm growth.

Because biofilms can also form on implanted medical devices, such as pacemakers and synthetic joints, effective treatments against biofilms could eliminate the need for costly and risky replacement surgeries and the expense of curing Hospital Acquired Infections. A familiar biofilm is the dental plaque that forms on teeth between brushings, but biofilms can form almost anywhere given the right conditions. When disease-causing bacteria establish a biofilm on sensitive equipment, it can be impossible to sterilize the devices, raising rates of infection and necessitating expensive replacements.

Hospital-associated infections cause considerable morbidity and mortality, and are expensive to treat. Organisms causing these infections can be sourced from the inanimate environment around a patient. These organisms are difficult to eradicate from the environment because they reside in dry surface biofilms.

One article describes a study wherein biofilms were present on several dry hospital surfaces in an Intensive Care Unit (ICU). (See "Biofilms make the hospital environment far from 'inanimate'", June 30,2015, Jon Otter, http://reflectionsipc.com/2015/06/30/biofilms-make-the-hospital-environment-far-from-inanimate/)

The study described samples that were taken of patient bedding (n = 11), patient surrounds (n = 19), and fixed furnishings in = 14) from an ICU. Exemplary locations that were tested in the ICU include: mattresses, pillows, pillow slips, curtains, videos, floors, and Velcro. Each sample was cultured using a conventional approach, tested for the presence of S. aureus by PCR, and examined for the presence of biofilm under electron microscopy. 18 samples underwent a live/dead microscopy technique 12 months after sample collection. In addition, 15 samples were pyrosequenced to evaluate their "environmentome".

Biofilm was detected on 41/44 (93%) of the samples. More surprisingly, all 18 of the samples evaluated using live/dead microscopy contained live bacteria 12 months after sample collection. Furthermore, Methicillin-resistant Staphylococcus aureus (MRSA) was cultured from 18% of the samples, Extended-Spectrum Beta-lactamase (ESBL) from 11% of the samples, Vancomycin-resistant Enterococcus (VRE) from 8% of the samples, and 50% of the samples gave a positive signal for S. aureus by polymerase chain reaction (PCR).

These results strongly illustrate the failure of conventional methods to remove biofilms from surfaces. One important aspect of this study is that samples were collected immediately after a two-step cleaning and disinfection protocol: a cleaning step using a detergent, followed by a disinfection step using bleach. These results clearly show that conventional methods do not eliminate biofilms. This is because biofilms provide physical protection from cleaning agents making microbes less susceptible to disinfectants by a factor of, for example, 10 to 1,000 fold. This study shows that daily cleaning of an ICU with existing products does little more than support the existence and propagation of these dangerous biofilms, as shown by the presence of live bacteria after 12 months.

Another study also showed that ICU surfaces are contaminated by multidrug-resistant bacteria present in biofilms (Hu, H., et al., The Journal of Hospital Infection, DOI:http/dx.doi.org/0.1016/j.jhin.2015.05.016).

The intensive care unit (ICU) of a hospital was decommissioned and the opportunity to destructively sample clinical surfaces was taken in order to investigate whether multidrug-resistant organisms (MDROs) had survived the decommissioning process and whether they were present in biofilms.

The ICU had two 'terminal cleans' with 500 ppm free chlorine solution; items from bedding, surrounds, and furnishings were then sampled with cutting implements. Sections were sonicated in tryptone soya broth and inoculated on to chromogenic plates to demonstrate MDROs, which were confirmed with the Vitek2 system. Genomic DNA was extracted directly from ICU samples, and subjected to polymerase chain reaction (PCR) for femA to detect Staphylococcus aureus and the microbiome was analyzed by bacterial tag-encoded FLX amplicon pyrosequencing. Confocal laser scanning microscopy (CLSM) and scanning electron microscopy (SEM) were performed on environmental samples.

Multidrug-resistant bacteria were cultured from 52% (23/44) of samples cultured. S. aureus PCR was positive in 50% of the samples. Biofilm was demonstrated in 93% (41/44) of samples by CLSM and/or SEM. Pyrosequencing demonstrated that the biofilms were polymicrobial and contained species that had multidrug-resistant strains.

Dry surface biofilms containing MDROs were found on ICU surfaces despite terminal cleaning with a chlorine solution.

Scanning electron micrographs of biofilms showed the following contaminated surfaces in the ICU; a privacy curtain; the ward entry door showed coccoid bacteria embedded in a thick amorphous extracellular polymeric substance (EPS); a mattress showing biofilm containing bacteria of various morphologies including rod-shaped organisms; a wire clip for holding patients' notes showed dense EPS with embedded coccoid bacteria.

Confocal laser scanning micrograph of biofilms stained with BacLight Live/Dead stain showed the presence of live bacteria and dead bacteria on dry hospital surfaces. The staining occurred after 12 months of storing the sample at room temperature. Live bacteria were found on: a storage box used to hold sterile supplies of single-use patient equipment; this surface cultured positive for Staphylococcus aureus. Live bacteria were found on: the ward entry door; this surface cultured positive for S. aureus by polymerase chain reaction.

### ROLE OF OLIGORIBONUCLEASES IN BIOFILM

Oligoribonucleases can be added to any of stock solutions disclosed herein (TABLE 2), or to a diluted version (TABLE 7) of a stock solution.

One recent study identified an enzyme that shuts down the signals that bacteria use to form a biofilm. This study is described in, "Oligoribonuclease is the primary degradative enzyme for pGpG in Pseudomonas aeruginosa that is required for cyclic-di-GMP turnover," Orr, M., et. al., published August 24, 2015 in the Early Online Edition of the Proceedings of the National Academy of Sciences.

A signaling molecule called Cyclic-di-GMP, also known as c-di-GMP activates biofilm formation. Many species of disease-causing bacteria use c-di-GMP to signal the formation of biofilms, including Escherichia coli, Salmonella enterica and Vibrio cholerae.

Orr and her colleagues identified a molecule that "turns off" the biofilm signaling process. The molecule is an enzyme called oligoribonuclease, and much like c-di-GMP, oligoribonuclease is also common among disease-causing bacterial species.

The team studied the process in the bacteria Pseudomonas aeruginosa, a common species known to cause infections in hospital patients. But because of the genetic and physiological similarities between P. aeruginosa and other infectious species, the researchers believe that oligoribonuclease serves the same function across a wide variety of bacteria.

The team found that oligoribonuclease is necessary for the second of a two-step process. The first step is the conversion of c-di-GMP into an intermediate molecule called pGpG, and the second step is breaking apart of pGpG by oligoribonuclease, resulting in the signaling pathway being shut down.

### MEASUREMENT OF ADENOSINE TRIPHOSPHATE

Adenosine triphosphate (ATP) is a nucleoside triphosphate used in cells as a coenzyme often called the "molecular unit of currency" of intracellular energy transfer. ATP is a molecule found in and around living cells, and as such it gives a direct measure of biological concentration and health. All organic matter (living or once-living) contains ATP, including food, bacteria, mold and other microorganisms. ATP is quantified by measuring the light produced through its reaction with the naturally occurring firefly enzyme luciferase using a luminometer. The amount of light produced is directly proportional to the amount of ATP present in the sample.

The detection of ATP on a surface therefore indicates the presence of biological matter that may not otherwise be visible to the eye. In industries where hygiene control is crucial, ATP testing is an excellent tool for detecting and measuring biological matter on surfaces and in water.

Hospitals are using ATP-based sanitation monitoring systems to detect and measure ATP on surfaces as a method of ensuring the effectiveness of their hospital's sanitation efforts (for example, as described in Kyriakides, A.L. and Patel, P.D. (1991) Rapid hygiene monitoring using ATP bioluminescence, Bioluminescence and Chemiluminescence: Current Status; 519 522. John Wiley and Sons, Chichester). Farmers can also use ATP-based sanitation monitoring systems to detect and measure ATP on surfaces as a method of ensuring the effectiveness of their facilities' sanitation efforts. The amount of ATP detected, and where this ATP was detected, indicates areas and items in the healthcare setting or livestock facility that may need to be recleaned, and the possible need for improvement in a healthcare facility's or livestock facility's cleaning protocols.

Just because an environmental surface looks clean does not mean it is clean. Microorganisms, biofilm and other biological residues are not visible to the unaided eye thus rendering the current visual inspection method for cleaning monitoring inadequate to the task. Adenosine triphosphate (ATP), has been found to be a sensitive indicator molecule for the presence of biological residues due to its universal presence in all living cells (microbes, animal, and plant cells). An increase in "dirt" (biological residues) on a surface results in an increase in the amount of ATP present on that surface making ATP an effective marker for the assessment of the hygienic status of an environmental surface.

It is well understood by one of skill in the art, that the higher the ATP reading, the more likely the surface, water, space, or air space will support pathogenic colonization and growth. In other words, the higher the ATP reading the higher the probability of the presence of pathogenic biofilm. Studies have shown that there is a direct correlation between ATP readings and microbiological plate assay results (for example, streaking of a sample on an agar plate) regarding the presence or absence of pathogenic biofilm.

Collecting ATP from a contaminated surface can be done by swabbing an area of consistent size (for example, four square inches). The amount of ATP present on the swab is a quantitative measurement of the cleanliness of the surface tested. To determine the hygienic status of an environmental surface one must measure the amount of ATP present on the swab. Directly measuring the concentration of ATP on the swab requires extensive training, sophisticated equipment and time. It is therefore easier and faster to determine the amount of ATP on a swab indirectly. There are many different methods that are used for ATP determination, but the most successful technique is the bioluminescent method because of its sensitivity and wide dynamic range. One can look to nature for examples of how various organisms like fish, bacteria, snails and fungi generate light through various bioluminescent processes. One of nature's most efficient and well-studied light-generating reactions is the ATP bioluminescence system found in Photinus pyralis, the firefly (see Gould, S.J. and Subramani, S. (1988) Firefly luciferase as a tool in molecular and cell biology. Anal. Biochem. 175 (1): 5-13.)

The ATP collected on the swab is used to generate a light signal using the luciferin-luciferase system described above. Under optimum conditions the amount of ATP is directly proportional to the amount of light emitted. A Relative Light Unit (RLU) is not a standardized unit of measurement like inches or centimeters. Because different ATP monitoring systems have different outputs, sensitivities, reagent formulations and light detection systems the RLU scales are different for each system. Each manufacturer sets their own value for 1 light unit and all measurements are made relative to that value. Larger RLU values do not indicate that a system is more sensitive. Because each manufacturer uses a different scale one must be careful not to use RLU values to compare different ATP monitoring systems.

The number and types of bacteria present on a surface is normally assessed using various microbiological methods. Viable plate counts are used to assess the number of living bacteria present on a surface where the results are expressed in colony forming units (CFUs) per area of surface tested. Certain devices detect ATP from bacteria in addition to all other biological sources of ATP and therefore are not measuring the numbers of bacteria (CPUs) but a measure of cleanliness (RLUs). Because CFU and RLU values are determined using different test methods and measure different substances, one would not expect RLU values to consistently correlate to CFUs when testing an environmental surface. ATP hygiene monitoring provides a measurement of the direct risks resulting from high levels of microorganisms, plus the indirect risks resulting from organic residues that can protect and provide a source of nutrients to microorganisms.

### EXEMPLARY DEVICES FOR MEASURING ATP

Any ATP detection device (or method) can be used with the methods disclosed herein. Exemplary ATP detection devices such as Scigiene's EnSURE & SystemSURE Plus ATP Detection Systems use ATP bioluminescence technology to measure extremely low levels of organic residue. Other exemplary devices include Bio-Reveal SystemSURE PLUS ATP Measurement System (ATP Meter), and SystemSURE Accessories by American Screening Corporation. The Hygiena Luminometer with UltraSnap Swabs can be used in the methods described herein. Additional examples of luminometers are 3M Clean-Trace Luminometer NGi, Lucetta Luminometer AAL-1001 and Charm Sciences Novalum Luminometer.

Other exemplary methods to measure ATP are an ATP Assay Kit (Colorimetric/Fluorometric), Luminescent ATP Detection Assay Kit, ATP synthase Enzyme Activity Microplate Assay, or a luciferase-luciferin based assay.

### REDUCTION IN A PATHOGEN OR BIOFILM

A reduction in a pathogen or a biofilm can be determined by any method known to one of skill in the art. Exemplary methods include, a detection assay, an immunoassay, a bacterial plate assay, a plate assay, a microbial plate testing, visualization, a reduction in pathogen count, any molecular assay test designed for environmental samples, any pathogen detection system, DNA-based diagnostic methods such as polymerase chain reaction (PCR) amplification techniques, optical technologies, and microarray-based detection techniques. Numerous companies sell assays to test for a reduction in a pathogen, for example, RokaBiosciences (U.S.A.), Oxoid Listeria Rapid Test (OLRT) by ThermoFisher Scientific (U.S.A.), and PremierBiosoft, Inc. (U.S.A.).

Reduction of a pathogen can be determined by any method known to one of skill in the art. For example, reduction of a pathogen can be determined by measuring cell count by microbial assessment methods, measured in CFU per a specific area.

### EXAMPLES

The following examples are intended to provide illustrations of the application of the present disclosure.

### EXAMPLE 1: USE OF M+ TO TREAT LISTERIA MONOCYTOGENES

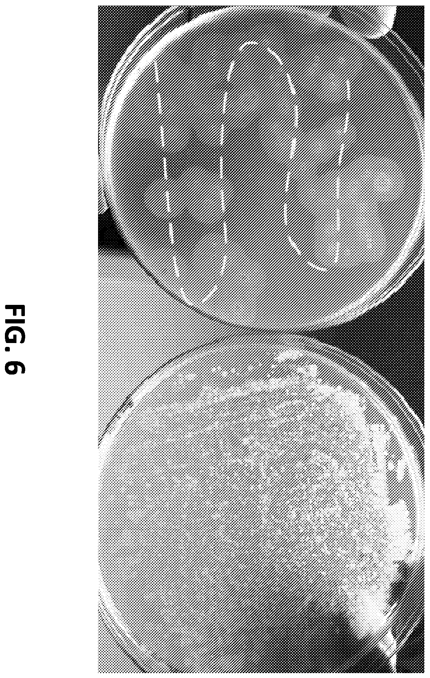

FIG. 6 is a photograph of two petri dishes, both inoculated with listeria monocytogenes ("listeria") from the same culture and with identical amounts. The petri dish on the left is a control. The petri dish on the right was streaked with 3 drops approximately 0.1 ml) of M+ at a concentration of 1:2 parts water. Both petri dishes were incubated for 24 hours at 35 degrees C. The photograph in FIG. 6 shows the two slides after the 24-hour incubation. The petri dish on the left shows that listeria is still present, as shown by the round circular growths. The petri dish on the right is covered with the probiotics present in M+, showing the motility of the bacteria as it completely covers the surface. Each of the petri dishes were then tested for the presence of living and dead cellular remains of listeria, using Biomedix Listeria Assay Solution. The control petri dish on the left contained live listeria. The petri dish on the right did not have any indication of either living or dead listeria cells. In the petri dish on the right, the listeria were inhibited, died, and were consumed by the probiotics in M+.

### EXAMPLE 2: REMOVAL OF BIOFILM FROM STAIRS

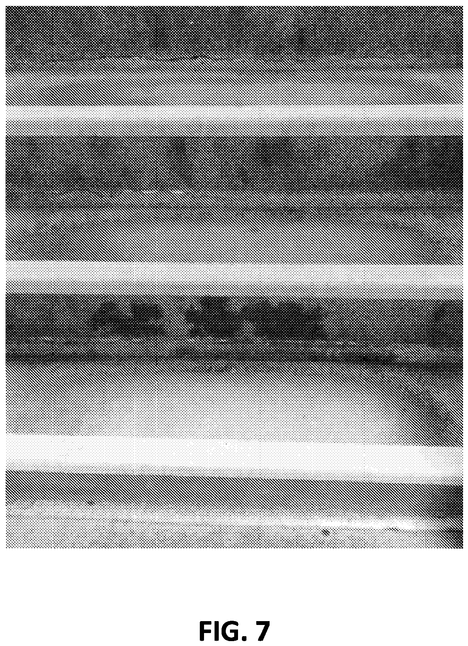

Shown in FIG. 7 are the first three steps of a wooden staircase adjacent to a concrete floor that was cleaned with B1+ diluted 1:20. Shoes tracked the B1+ up onto each ascending step from the concrete floor.

The photograph in FIG. 7 shows the efficacy of B1+ to remove or reduce biofilm. At the bottom of the photograph is a concrete floor that is located at the entry of a set of wooden stairs ascending to an employee break room in a poultry processing facility. The original intention was to remove the grease, dirt, and biofilm from the concrete walkway using B1+, because the stairs are wooden there was no attempt to wash them. Each of the stairs was uniformly black at the time the walkway was first cleaned with B1+. The semioval, lighter colored areas of the stairs are where the employees walked as they ascended the stairs. The action of walking across the concrete floor resulted in tracking residual probiotic bacteria from the B1+ onto the stairs. Once the bacteria were tracked onto the stairs it broke down the biofilm as evidenced by the lighter semioval area on each tread (the top surface of a step). As less and less probiotic was tracked up each successive stair, less of the biofilm was removed.

### EXAMPLE 3: COMPARISON OF CF+ WITH COMMERCIALLY AVAILABLE ECOLAB FLOOR CLEANER (ECOLAB; USA)

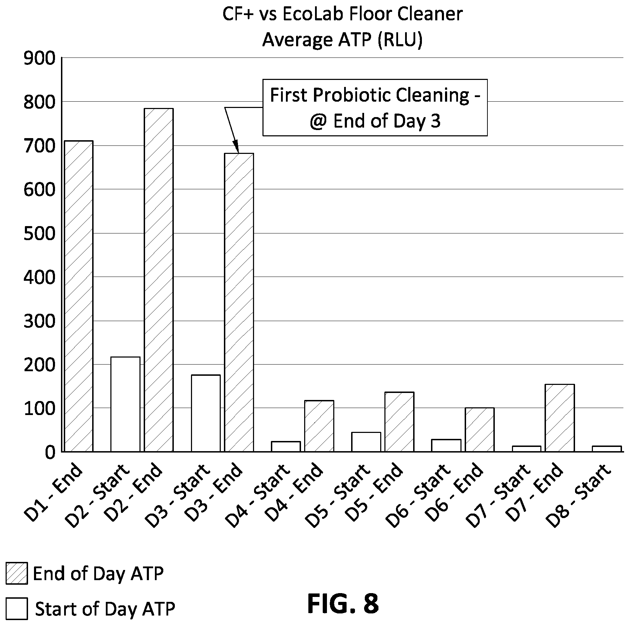

FIG. 8 compares the efficacy of CF+ diluted 1:64 with that of EcoLab Floor Cleaner, as determined by measuring ATP in RLU (relative light units). The y-axis represents average RLU and the x-axis represents days. 12 ATP readings per day were taken over an 8-day period. Each reading was taken at the same location on a floor of a store, for traffic consistency. Readings were taken at the end of each business day before floor cleaning and immediately before the store was opened at the start of the next business day. Each set of 12 readings was averaged. The results are shown in FIG. 8. EcoLab Floor Cleaner was used to clean the floor at the end of day 1 and at the end of day 2 (D1-End and D2-End). The first time CF+ was used was at the end of day 3 (D3-End).

In FIG. 8, the first three "End of Day" ATP (RLU) readings (D1-End, D2-End, and D3-End) using EcoLab Floor Cleaner alone, showed an average ATP reading of greater than 700 RLU. The average End of Day ATP (RLU) reading after cleaning with CF+ (D4-End, D5-End, D6-End, and D7-End,) was less than 125 RLU. Using EcoLab Floor Cleaner, the average ATP (RLU) reading taken at the "Start" of each of the 2 business days (D1-Start, D2-Start) was 195 RLU. Using CF+, the average ATP (RLU) reading taken at the "Start" of each of the 5 business days (D4-Start, D5-Start, D6-Start, D7-Start, and D8-Start) was 18 RLU. The efficacy of CF+ is clearly demonstrated by the reduction in ATP at both the start and end of the business days 4 to 8.

### EXAMPLE 4: COMPARISON OF POULT MANURE DROPPINGS

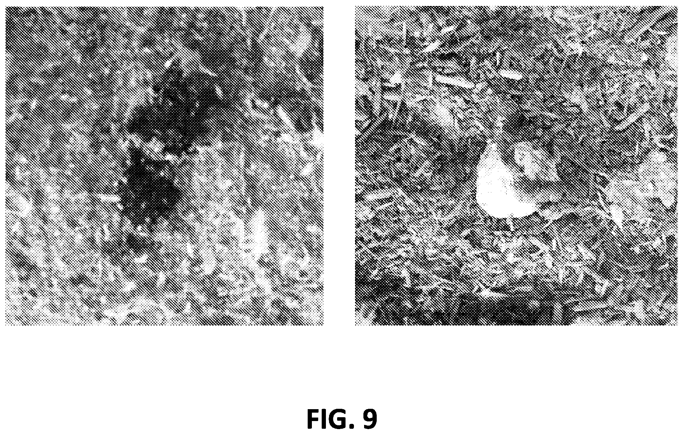

FIG. 9 shows two photographs of typical manure droppings from 20-day-old poults in a brooder barn. The photograph on the left and the photograph on the right are typical manure droppings from the brooder barn of 20-day-old poults. In the photograph on the left, the black runny liquid appearance of the manure dropping is indicative of diarrhea and an unhealthy gut (gastrointestinal tract). After the photo on the left was taken, the water going into the brooder house (that the poults ingested) was inoculated with W+ on a constant basis at a dilution of 1:10,000. The photo on the right was taken 48 hours later, and dramatically shows the change in the manure dropping, from a black runny liquid to a white semi-solid form, indicating a healthy gut.

### EXAMPLE 5: EFFECT OF A1+, M+, AND WATER PRODUCT W+ IN A HATCHERY

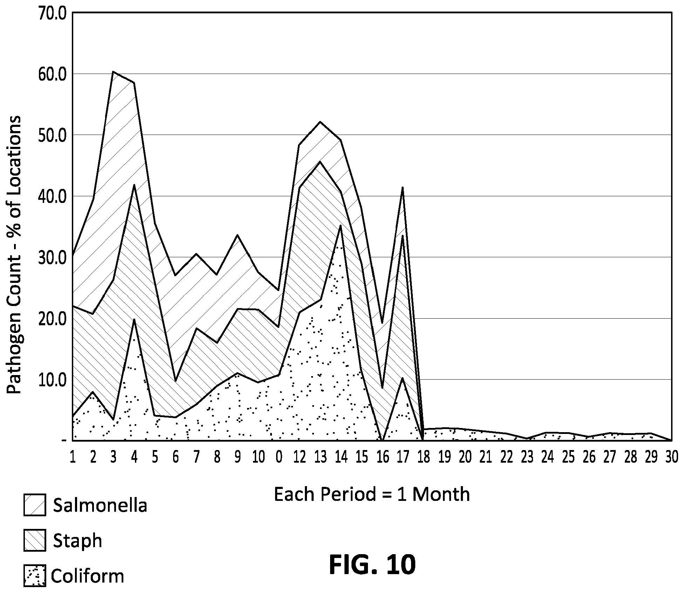

A poultry farmer cleaned and sanitized his poultry hatchery using a traditional cleaning and sanitizing protocol. After the cleaning and sanitizing, the ATP count still showed high levels of pathogens (see FIG. 10, weeks 1 to 17). FIG. 10 shows that by using several of the compositions (A1+ diluted 1:64, M+ diluted 1:2, and W+ diluted 1:10,000), the exposure of his poultry to pathogens was reduced up to 99%. Not only was the presence of pathogens almost entirely eliminated, but also the incredibly low levels were maintained for several months (weeks 19-30).

Salmonella, S. aureus, and coliforms are ubiquitous in poultry hatcheries. The graph in FIG. 10 shows the percentage of locations (surfaces) within a hatchery that had the presence of each of the pathogens. The presence of each of the pathogens was determined by microbiological plate testing (for example, streaking a sample onto an agar plate). The y-axis represents pathogen count (% of locations), and the x-axis represents a 30-month period, in one-month increments.

Products traditionally used in the industry, such as surfactants, chlorine, quaternary, and hydrogen peroxide, in addition to misting dangerous products, such as formaldehyde in areas such as the cold storage egg room, were used from Week 1 to Week 18. The hatchery was cleaned on a daily basis at the end of each work day. The period of Week 1 to Week 18 showed that an incredibly high percentage (for example, between 10% to 60%) of all of the surfaces tested had high levels of all three pathogens.

Beginning Week 18, the prior traditional protocol (described above) was stopped and the hatchery used only probiotic cleaner (A1+), spray (M+), and water product (W+)(A1+ diluted 1:64, M+ diluted 1:2, and W+ diluted 1:10,000). The A1+ cleaner was used to clean all of the same surfaces (as described above), then after that, all of the surfaces were sprayed with M+. In addition, the formaldehyde was replaced with M+ for misting in the cold-storage egg room. Also, a normal disinfectant that was used in boot-wash trays at each door between departments in the hatchery, was replaced with A1+. In addition, W+ was introduced at the entry point of the main water supply for the hatchery. The results are startling, clearly showing that a change from the traditional protocol to the use of compositions (A1+), spray (M+) and water product (W+), results in a drastic reduction of salmonella (0%) and S. aureus (0%) in the hatchery, and a reduction of coliforms from as high as 60% during the first 18 weeks to a level that did not exceed 2% after Week 18. Furthermore, the drastic reduction of the presence of all three pathogens continued from Week 18 to Week 30.

### EXAMPLE 6: EFFICACY OF C+ USING A BOOT TEST

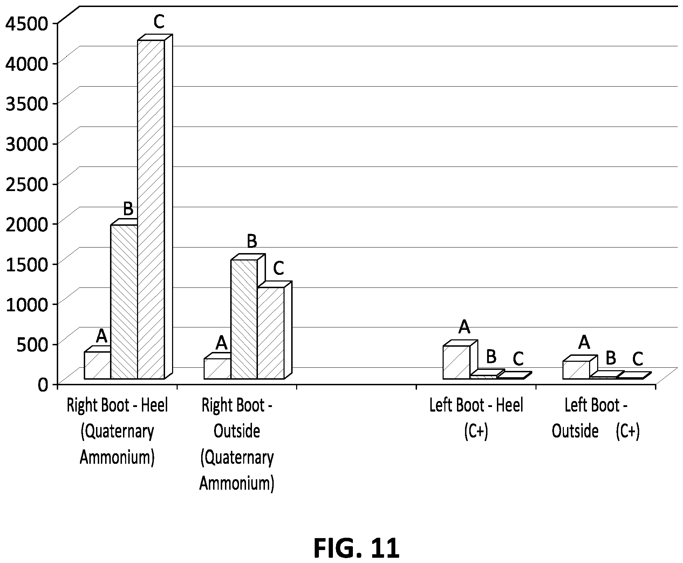

FIG. 11 shows the efficacy of C+ cleaner to remove and prevent biofilm, as shown by long term ATP reduction. The y-axis represents ATP (RLU). The x-axis represents a left and a right boot, two different locations on the bottom of each boot (the heel and the outside portion), and two different cleaners, quaternary ammonium and C+ at a dilution of 1:30.

(A) represents ATP measurements taken from the bottom (the heel and the outside) of a left and a right boot that were cleaned with either quaternary ammonium or C+. (B) represents ATP measurements taken from the bottom (the heel and the outside) of the same left and a right boot immediately after walking the floor of an active turkey grow out barn with the boots. (C) represents ATP measurements taken from the bottom (the heel and the outside) of the same left and a right boot after the boots have been left sitting undisturbed for 16 hours after walking the floor of the barn. For each measurement, the ATP measurements were taken from similar locations on both the right boot and the left boot. For example, a measurement was taken from approximately the same location on the heel of the left boot and the heel of the right boot.

Results of the experiment are shown below in Table 9 and FIG. 11. Immediately following cleaning with quaternary ammonium (right boot) and C+ cleaner (left boot), the ATP readings for both products were approximately the same as shown by (A) in FIG. 11. After walking the barn (B), the ATP measurement of the right boot, cleaned with quaternary ammonium, had increased dramatically, averaging 1,710 RLU. In stark contrast, the left boot, cleaned with C+, had decreased to an average of 36 RLU. After each boot was left undisturbed for 16 hours (C), the ATP reading on the boot cleaned with quaternary ammonium had increased to an average of 2,692 RLU compared to an average ATP of 21 RLU for the boot cleaned with C+. The data obtained from the experiment is presented below.

**TABLE 8: ATP MEASUREMENTS TAKEN FROM RIGHT AND LEFT BOOT**

| Boot and Disinfectant | (A) ATP After Cleaning - Before Walking the Barn | (B) ATP tested right after walking barn and cleaning | (C) ATP tested 16 hours after (B) |
|---|---|---|---|
| Right Boot-Heel Quaternary | 347 | 1926 | 4228 |
| Right Boot-Outside Quaternary | 259 | 1494 | 1156 |
| Left Boot-Heel C+ | 421 | 42 | 23 |
| Left Boot-Outside C+ | 230 | 29 | 19 |

### EXAMPLE 7: USE OF W+ TO CLEAN SURFACES OF WATER LINES

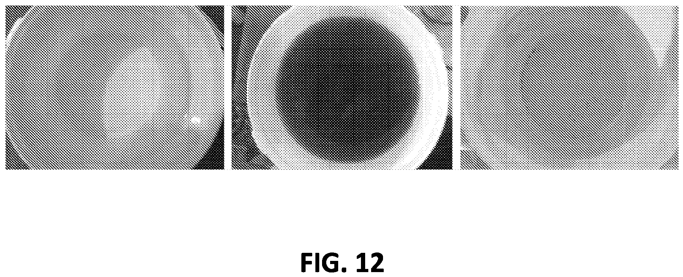

The three panels of FIG. 12 are photographs of a top-view of three buckets. Each bucket contains an equal amount of water taken at different times from a water line in a poultry (turkey) farm. The water flowing through the water line is the water that the turkeys consume (drink).

A cleaning protocol typical for poultry (and swine livestock) growers was used to clean the water line. This protocol is as follows. Between turkey flocks, a water line is treated with Proxy Blast (35% hydrogen peroxide)(Proxy-Clean Products; U.S.A.), or similar products that may be hydrogen peroxide based (CID-2000), paracidic acid based, or very concentrated chlorine based products (for example, sodium hypochlorite or chlorine-dioxide). The Proxy Blast is left to sit in the water line for approximately three days. During the growing period of the flock, the water line is also constantly flushed with 5 ppm of chlorine dioxide. Both of these actions are an effort to eliminate and prevent the growth of biofilm in the water line.

The left hand panel shows a top view of a bucket containing water that was removed from the water line after the three-day treatment with Proxy Blast, but prior to cleaning with C+. The water removed from the line appears to be clean giving the perception that the line is clean and no biofilm is present. C+ at a dilution of 1:100 was then added to the water line and allowed to remain undisturbed for five days. The middle panel shows water removed from the water line after the five-day treatment with C+. The middle panel shows the presence of dark colored biofilm residue. This biofilm was adhering to the surface of the water line despite the previous attempts to remove it with Proxy Blast and prevent its formation using chlorine dioxide. After cleaning and flushing the water line, W+ was added at a dilution of 1: 10,000, allowed to stand over-night, and drained the next morning (the right hand panel). The right hand panel shows water obtained from the water line after treatment with W+. The right hand panel shows additional small dark colored particles of biofilm that were released from the water line after treatment with W+.

### EXAMPLE 8: EFFECT OF W+ ON TURKEY MORTALITY

Chlorine dioxide is widely used to treat water lines in livestock growing facilities, and has been used for many years. Chlorine dioxide treatment was believed to be an effective treatment for the prevention of biofilm, and not to be harmful to the livestock. The water that flows through the water lines is consumed by the livestock.

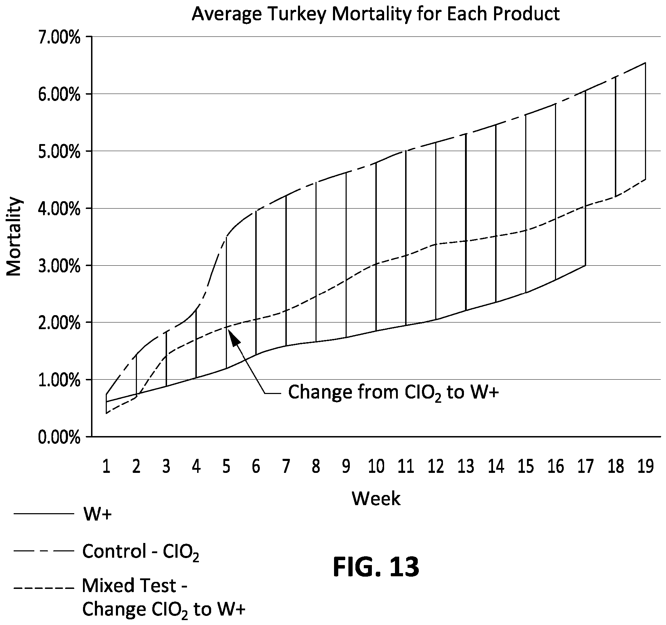

FIG. 13 clearly shows that consumption of water with chlorine dioxide by 50,000 turkeys results in a much higher mortality rate as compared to water with W+. The mortality of the flock of 50,000 turkeys that consumed water with chlorine dioxide was 6.5% as compared to 3% for a flock of 50,000 turkeys consuming water with W+. The y-axis of FIG. 13 represents the cumulative total mortality (%), and the x-axis represents a 19-week period during which the turkeys were observed. The top dotted line represents a flock (of 50,000 turkeys) with chlorine dioxide added to their water. The middle dotted line represents a flock (of 50,000 turkeys) that for four weeks consumed water treated with chlorine dioxide (5 ppm). After four weeks, the water additive was changed to W+ diluted 1: 10,000 (note the reduced slope of the mortality curve at week 5). The bottom solid line represents a flock (of 50,000 turkeys) that was fed water with W+. The data for this experiment is shown below in Table 9.

**TABLE 9: MORTALITY CUMULATIVE TOTAL PER GROUP**

| **Week** | **W+** | **Chlorine Dioxide** | **Change Chlorine Dioxide to W+ at 4 weeks** |
|---|---|---|---|
| 1 | 0.61% | 0.74% | 0.41 % |
| 2 | 0.75% | 1.45% | 0.69% |
| 3 | 0.88% | 1.84% | 1.42% |
| 4 | 1.04% | 2.15% | 1.72% |
| 5 | 1.19% | 3.52% | 1.93% |
| 6 | 1.44% | 3.97% | 2.05% |
| 7 | 1.60% | 4.22% | 2.20% |
| 8 | 1.66% | 4.46% | 2.45% |
| 9 | 1.73% | 4.62% | 2.74% |
| 10 | 1.85% | 4.79% | 3.03% |
| 11 | 1.95% | 5.01% | 3.17% |
| 12 | 2.05% | 5.14% | 3.37% |
| 13 | 2.20% | 5.29% | 3.43% |
| 14 | 2.35% | 5.46% | 3.51% |
| 15 | 2.53% | 5.63% | 3.61% |
| 16 | 2.76% | 5.82% | 3.81% |
| 17 | 3.00% | 6.05% | 4.04% |
| 18 | | 6.29% | 4.20% |
| 19 | | 6.54% | 4.51% |
| 20 | | 6.84% | 5.01% |
| 21 | | 6.74% | 5.75% |

### EXAMPLE 9: EFFECT OF W+ ON WEIGHT GAIN OF TURKEYS

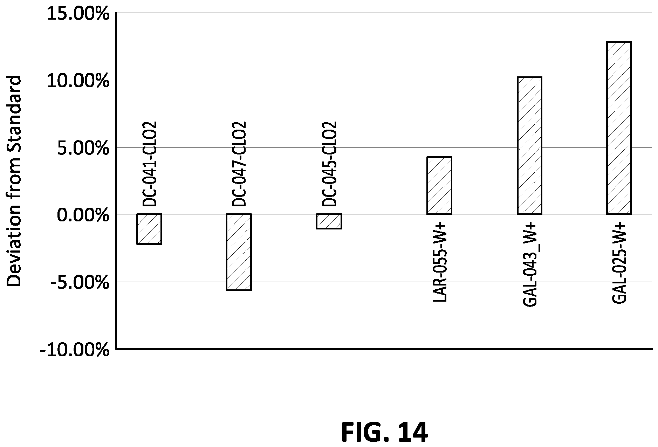

A common practice among commercial turkey growing facilities is to compare the weight gain of their flocks against a standard that is based on the performance of the grower's previous flocks. The grower has a standard for each barn, or group of banns, based on their recorded history. FIG. 14 shows the efficacy of W+ in the water supply of a flock when compared to growing standards (Std). The flocks in the barns being fed W+ in their water (Barns-055, Barn-043, and Barn-025) exceeded standard weights by 11.7%, while the barns where the turkeys were fed water with chlorine dioxide (Barns-041, Barn-047, and Barn-(45) were -7.6% compared to standard weights. The y-axis represents % deviation from a standard (Std)(average for each barn), and the x-axis represents data obtained from turkeys in six different barns (approximately 17,000 turkeys per barn).

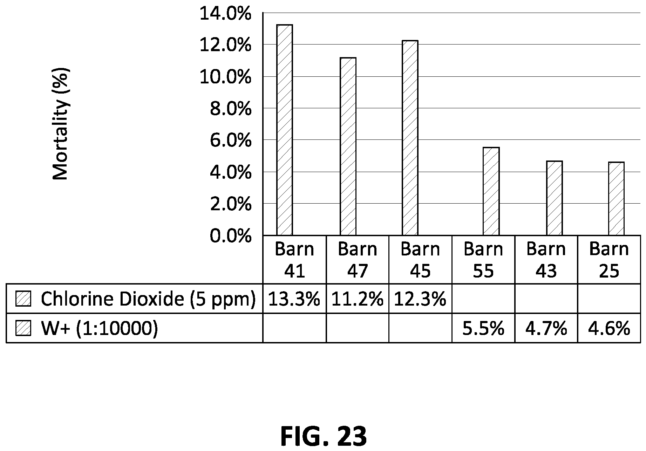

FIG. 14 shows the growth rate of the turkeys as compared to the standard; the turkeys that consumed W+ in their drinking water had a higher average weight (deviation from Std) than turkeys that consumed chlorine dioxide in their water. In FIG. 23, the turkeys fed water with chlorine dioxide had a higher mortality rate (an average of 12.3%.) as compared to the flock fed water with W+ (an average of 4.9%).

### EXAMPLE 10:SWINE GUT HEALTH

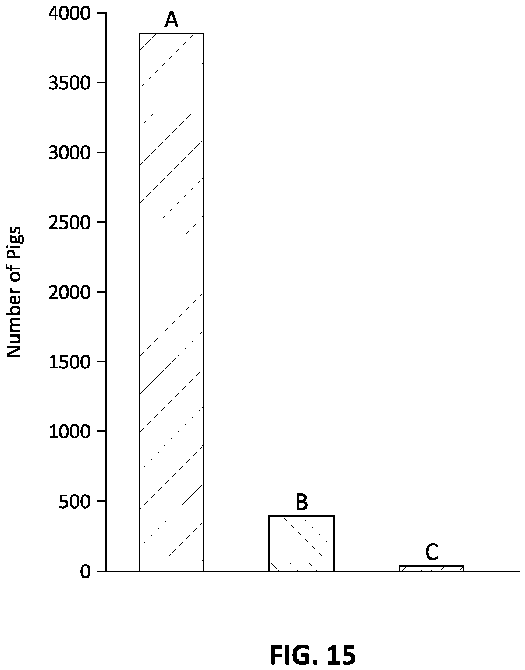

Scours (dysentery) is a common ailment in swine and is symptomatic of poor gut health (gastrointestinal tract health). Scours (for example, Clostridial scours) is usually caused by the presence of pathogenic bacteria in the gut. In FIG. 15, column (A) shows a total population of 3,850 swine on a test farm. Column (B) is the number of swine (397) that were infected with scours while on a standard water protocol of chlorine dioxide to prevent biofilm in the water lines. The addition of chlorine dioxide was stopped and W+ (at a dilution of 1 :6,000) was added to the water lines, the number of swine with scours was reduced to 23 (Column (C)), a 94% reduction in the number of swine with Clostridial scours.

### EXAMPLE 11: COMPARISON OF CO+ WITH NORMAL CLEANING AND DISINFECTION PROTOCOL - PROCESSING PLANT NO. 1

A poultry processing factory, Processing Plant 1, tested their Food Safety and Inspection Service (FSIS) approved cleaning and disinfection procedure against CO+ alone. Day one, the processing plant was cleaned using the FSIS cleaning and disinfectant protocol. 24 samples were taken from different surfaces (for example, stainless steel troughs, conveyors, processing equipment, walls, and floors) throughout the processing plant, and an ATP luminometer (measuring RLU) was used to test the soil and fat remaining on the surfaces. Day two, CO+ was used to clean 24 similar surfaces within the processing plant, and no disinfectant was used. Samples were taken and an ATP luminometer (measuring RLU) was used to test the soil and fat remaining on the surfaces.

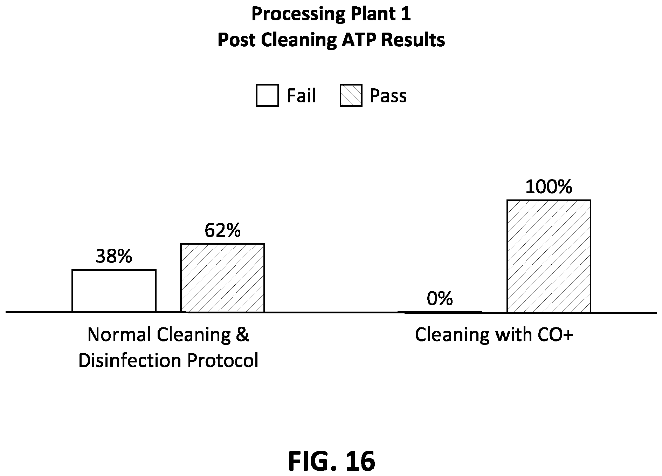

The results of the FSIS approved cleaning and disinfection protocol are represented by the two left hand columns of FIG. 16. The results of the CO+ protocol are represented by the two right hand columns of FIG. 16. All surfaces that were tested were compared against a standard of 15 RLU as the maximum acceptable value. In regards to the FSIS approved cleaning and disinfection protocol, 62% of surfaces passed and 38% of the surfaces failed. The same test using CO+ resulted in 100% of the surfaces tested passing and 0 failures. In FIG. 16, the dark bars represent RLU values that failed (above 15 RLU), the light bars represents RLU values that passed (below 15 RLU).

### EXAMPLE 12: COMPARISON OF CO+ WITH NORMAL CLEANING AND DISINFECTION PROTOCOL - PROCESSING PLANT NO. 2

A poultry processing factory, Processing Plant 2, tested their Food Safety and Inspection Service (FSIS) approved cleaning and disinfection procedure against CO+ alone.

Day one to five, the processing plant was cleaned using the FSIS cleaning and disinfectant protocol. Day six to 10, CO+ was used in place of the FSIS protocol, and no disinfectant was used. Day 11, the plant returned to the previous FSIS protocol. Each day 24 samples were taken from different surfaces (for example, stainless steel troughs, conveyors, processing equipment, walls, and floors) throughout the processing plant, and an ATP luminometer (measuring RLU) was used to test the soil and fat remaining on the surfaces.

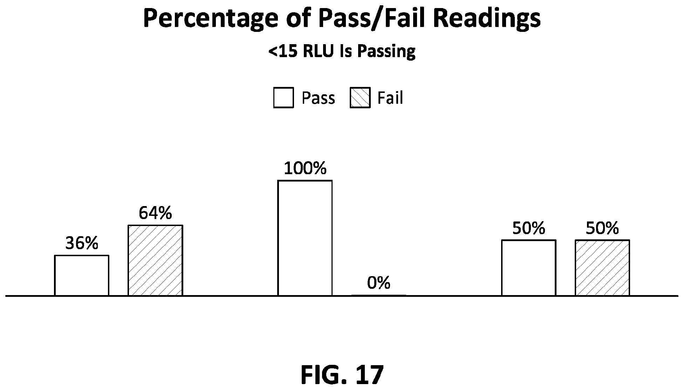

The results of the FSIS approved cleaning and disinfection protocol are represented by the two left hand columns of FIG. 17 and the two right hand columns of FIG. 17. The results of the CO+ protocol are represented by the two middle columns of FIG. 17. All surfaces that were tested were compared against a standard of 15 RLU as the maximum acceptable value.

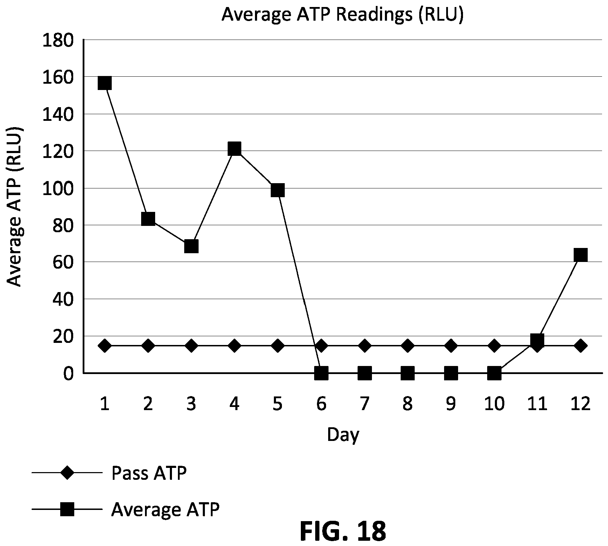

FIG. 18 is a graph of the data represented in FIG. 17. This graph shows the average ATP readings for each day during the test. Day 1 through Day 5 the plant was cleaned and disinfected using the standard FSIS protocol. Starting on Day 6 through Day 10 the plant used CO+ in place of the FSIS protocol, but ran out of CO+ on Day 10. On Day 11, the plant had to return to the previous FSIS protocol. Note that on Day 11, CO+ still gave better results than the FSIS protocol.

In FIG. 17, the two left hand columns, using the FSIS approved cleaning and disinfection protocol, showed that 36% of surfaces passed and 64% of the surfaces failed, over a 6-day period (day 1 to day 6 on FIG. 18). The two middle columns show that by replacing the FSIS cleaning and disinfectant protocol with CO+, 100% of the surfaces passed and 0 failed, over a 5-day period immediately following (day 6 to 10 on FIG. 18). An additional 2-day period (day 11 and day 12 on FIG. 18) using the FSIS approved cleaning and disinfection protocol, is shown in the two right hand columns. The two right hand columns show 50% of surfaces passed and 50% of the surfaces failed over a 2-day period. In FIG. 17, the light bars represent RLU values that passed (below 15 RLU), the dark bars represents RLU values that failed (above 15 RLU).

### EXAMPLE 13: EFFICACY OF D+T128 ON TEATS OF DAIRY COWS

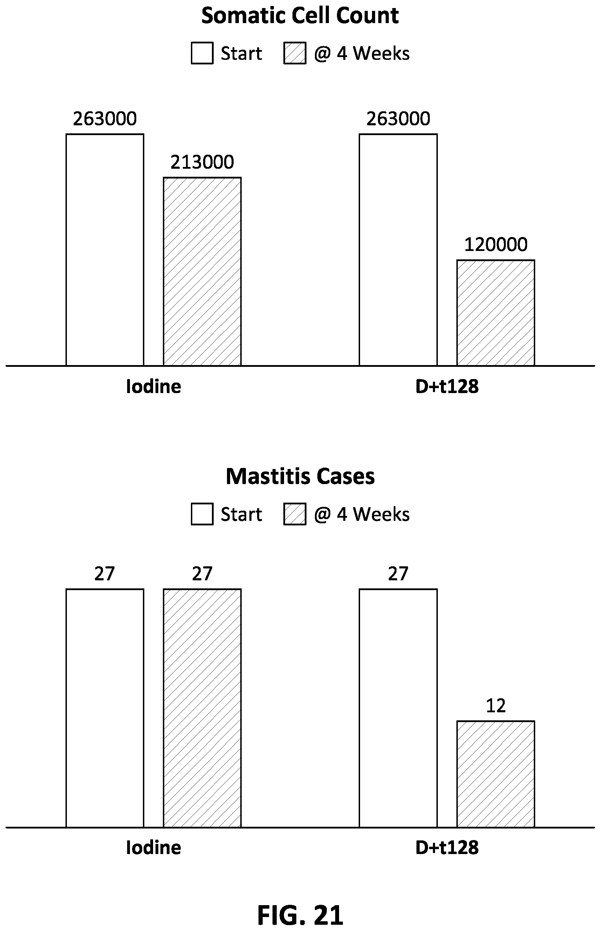

The efficacy of iodine versus D+t128 in reducing the presence of Mastitis was studied. FIG. 21 shows the results of the study. 210 lactating dairy cows were milked twice a day for four weeks. The somatic cell count (SCC) of the herd at the beginning of the test period was 263,000/ml, as shown in the two right-hand columns of the top panel of FIG. 21. The standard protocol using iodine was as follows: prior to each milking, the teats of the dairy cows were sprayed with 0.5% iodine. After each milking, the teats of the dairy cows were sprayed with 1.0% iodine. Mastitis was evident on 27 of the cows (see bottom panel of FIG. 21). Treatment with iodine was then stopped, and D+t128 was used to treat the teats of the cows. Prior to each milking, the teats of the dairy cows were sprayed with 1.25 ounces of D+t128 at a dilution of 1:128. After each milking, the teats of the dairy cows were sprayed with 1.25 ounces of D+t128 at a dilution of 1:128. At the end of the test period, the SSC had decreased from 263,000/ml to 120,000/ml in the herd treated with D+t128 (see two right-hand columns of the top panel of FIG. 21). The lower the SSC, the healthier the cow; the lower SSC reading correlates with the reduced number of cows with Mastitis. The number of cows with Mastitis had decreased from 27 to 12 (see two right-hand columns of bottom panel of FIG. 21). The SSC values are an average of all milk obtained from the 210 lactating cows. The decrease in the SSC count for the iodine treated cows (see two left-hand columns of top panel of FIG. 21) shows a natural seasonal reduction in SSC.

In the top panel, the y-axis represents somatic cell count, and the x-axis represents treatment with iodine or D+t128. Each pair of columns represents data obtained from the start (left column) and the finish (right column) of the 28-day test period. In the bottom panel, the y-axis represents the number of cows with Mastitis. The x-axis represents the start and finish of the 28-day test period.

### EXAMPLE 14: EFFICACY OF D+H100 OF HOOVES OF DAIRY COWS

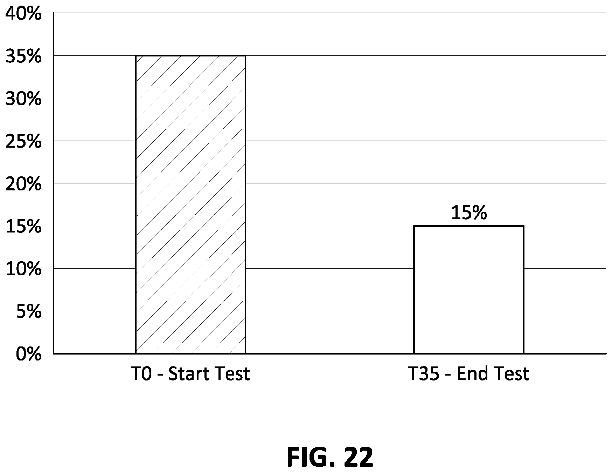

Applying D+h100 (diluted 1:100) onto a hoof of a dairy cow significantly reduced the presence of hairy warts, and decreased the presence of foot rot. These results are shown in FIG. 22. Copper-Sulphate is a commonly used treatment for cow hooves (another commonly used treatment is formaldehyde) and may be sprayed or used in a foot bath the cows walk through. There are significant environment and health issues with both of these treatments. Copper-Sulphate is contaminating fields, streams, and grain because it contaminates liquids and manure that is then spread onto fields. Formaldehyde is a known carcinogen and an environmental hazard. Additionally, to be effective against hairy wart and foot rot, formaldehyde must be used at a concentration that is lethal to cows if they should fall while traversing the foot bath, which happens on occasion.

The test period was 35 days long. A herd of 500 lactating cows had over 35% lameness at the start of the test period (T0 - Start Test), as shown in the left hand column of FIG. 22. In addition, 60% had swelling indicating new hairy wart formation (data not shown). The farm conditions were extremely dirty with some cows standing in six inches of manure. Prior to time 0, the hoof treatment used was an automated spray system spraying a solution of Copper-Sulphate twice per day as the cows exited the milking barn. The Copper-Sulfate solution was 25 lbs. of Copper-Sulfate diluted into 100 gallons of water. At time 0, the Copper-Sulphate used in the spray system was replaced by D+h100 diluted 1:100. The right hand column of FIG. 22 shows that the percentage of lame cows at the end of the test period decreased to 15%, day 35 (T35 - End Test); a reduction of 20% in herd lameness over the 35-day test. The efficacy of D+h100 to reduce hairy wart and foot rot infection is clear and the product is safe, environmentally friendly, and allows the elimination of products that contaminate the environment and are hazardous.

### EXAMPLE 15: HVAC- PERFORMANCE & BTU OUTPUT CASE STUDY

Cleaning heating, ventilation, and air conditioning (HVAC) coils is a more complicated task than was previously recognized. It is common practice to use nothing more than a garden hose with a standard water main pressure (for example, 50 psi to 80 psi) or a pressure washer (for example, at 200 psi to 1,000 psi) to spray the coils; for multi-row coils this does not clean the coils but rather compacts the detritus and bacteria into the center rows. HVAC coils in schools and commercial buildings are usually 6 rows to 12 rows deep with offset coils, and are tightly packed so it is difficult or impossible for air or water to pass between the coils once they become compacted with detritus and bacteria at their center. Consequently, multi-row coils do not have good air flow and, in addition, are heavily covered with biofilm.

HVAC coils are a natural biofilm producer - moisture is condensed from the air when the moisture in the air comes in contact with the colder metal surface of the coil. The condensed water then drips onto the coils below or to the down-wind side providing a constant flow of water containing planktonic bacteria that are picked up from the air. An HVAC coil closely represents the type of unit that is used in research labs to intentionally produce biofilm for research and studies.

The presence of biofilm on the coils is harmful to efficiency of the HVAC coils for several reasons. For example, detritus adheres more readily to biofilm than it does to clean metal, and the thermal transfer properties of biofilm are extremely bad (for example, 23% of that for Ferrous Oxide).

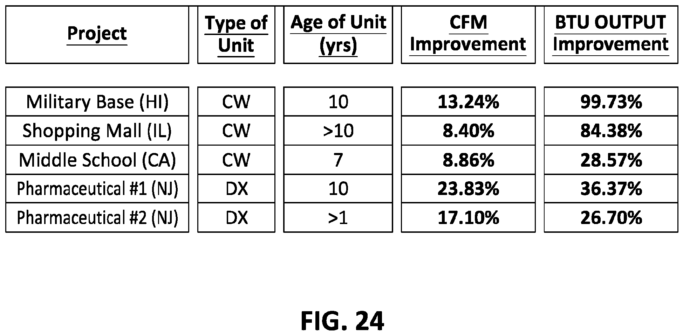

In each of the examples in the chart of FIG. 24 a protocol utilizing the AC-C and AC-S was used. After cleaning the coil of each of the cooling systems using high-pressure steam, AC-C (diluted 1:10) was pressure sprayed to the coils and allowed to remain for the time on the chart. Following cleaning with AC-C, the coils were sprayed with AC-S (diluted 1:2). For example, the Pharmaceutical #1 (NJ) Project used an AC-C dilution of 1:10 was foamed onto the coils and it remained on the coil 25 hours after which high-pressure steam was used to remove the contamination resident in the center of the coil rows. Following the high pressure steam cleaning with AC-C, the coils were sprayed with a fine mist of AC-S. The air flow through the coil, as measured in cubic feet per minute (CFM), was increased by 23.83%, and the net BTU, (British Thermal Units (BTU); a standard measurement of work) output was increased by 36.7%. The increase in BTU output is directly tied to a 36.7% reduction in the energy required to operate this equipment.

The AC products remove the detritus and other contaminants that impede air flow and remove and protect from biofilm reformation thus improving the heat transfer properties of the coils that reduces operating costs and makes the equipment last longer for both DX roof top systems and larger central CW (cold water) system.

### EXAMPLE 16: COMPARISON OF A1+ AND RETALIATE ON CLEANING VARIOUS SURFACES AT A MARINE BASE

ATP tests were carried out by cleaning personnel at a Marine Base. A1+ at a dilution of 1:100 was compared to "Retaliate" from Maintex Corp. (undiluted). The ATP results, shown in Table 10, are the average of four reading from the following four surfaces: women's shower floor; women's sink; men's shower floor; and men's door handle. Retaliate was used to clean all four surfaces on day 1 to day 6, A1+ was then used to clean the same surfaces on days 7 to day 13.

**TABLE 10**

| Day | Product | Average ATP |
|---|---|---|
| 1 | Retaliate | 155 |
| 2 | Retaliate | 127 |
| 3 | Retaliate | 145 |
| 4 | Retaliate | 299 |
| 5 | Retaliate | 194 |
| 6 | Retaliate | 168 |
| 7 | A1+ | 45 |
| 8 | A1+ | 7 |
| 9 | A1+ | 19 |
| 10 | A1+ | 15 |
| 11 | A1+ | 11 |
| 12 | A1+ | 16 |
| 13 | A1+ | 9 |

### EXAMPLE 17: COMPARISION OF A1+ AND ZEP ON CLEANING VARIOUS SURFACES AT A RAIL MAINTENANCE TERMINAL

ATP tests were carried out in passenger cars at a light rail maintenance terminal. The products tested were A1+ at a dilution of 1:100 and Zepynamic A II Surface Disinfectant (ZEP Corporation)(listed in Table 11 as "ZEP"). The ATP results shown in Table 11 are the average of the readings taken from the following six surfaces: barrier between seat and stair; door buttons; step hand rail; rail on back of seat; vertical hand rail on back of seat; and vertical hand rail. The surfaces cleaned with Zepynamic A II Surface Disinfectant had a 62% ATP reduction and the surfaces cleaned with A1+ had a 96% ATP reduction.

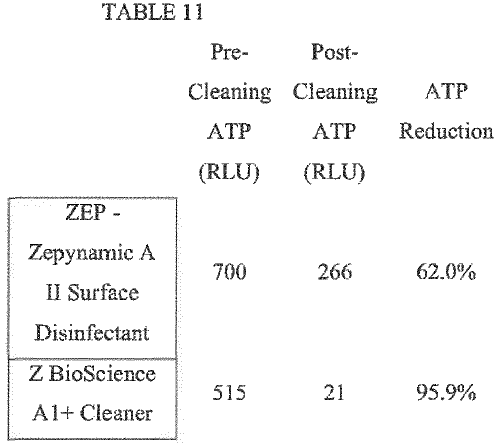

### EXAMPLE 18: COMPARISION OF C+ AND DUPONT VIRKON DISINFECTANT ON CLEANING CONTAMINATED SPECLNLAN TEST PANS.

ATP testing was carried out by a veterinarian at a Veterinary Medical Center. The products tested were C+ at a dilution of 1:30 and Dupont Virkon Disinfectant (Virkron Dupont) at a 2% solution. The ATP reduction results shown in Table 12 are the average of three readings taken from six aluminum specimen test pans for each product tested that were contaminated with swine feces. The contaminated pans cleaned with Virkon had a 92% ATP reduction at the time of testing but was at 89% after 48 hours. The contaminated pans cleaned with C+ had a 99.9% ATP reduction at the time of testing and was still at 99.5% after 48 hours.

**TABLE 12**

| | **10 min** | **24 hr** | **48 hr** |
|---|---|---|---|
| | **ATP** | **ATP** | **ATP** |
| Virkon % | | | |
| Reduction | 92.4% | 95.2% | 89.0% |
| C+ % Reduction | 99.9% | 99.8% | 99.5% |

### EXAMPLE 19: COMPARISION OF C+ AND TEKTROL DISINFECTANT ON CLEANING CONTAMINATED SURFACES

ATP testing was carried out by a veterinarian at a veterinary medical center. The products tested were C+ at a dilution of 1:30 and Tek-Trol Disinfectant (Tek Industries). The ATP results shown in Table 13 are the average of the readings taken from the following surfaces: water bar; vertical stall surface; and piglet side contact surface. The surfaces cleaned with Tek-Trol had a 59% ATP reduction. The surfaces cleaned with Z BioScience C+ had a 96% ATP reduction.

**TABLE 13**

| | Pre-Cleaning ATP (RLU) | Post-Cleaning ATP (RLU) | ATP Reduction |
|---|---|---|---|
| Tek-Trol Disinfectant | 6124 | 1752 | 71.4% |
| Z BioScience C+ Cleaner | 5186 | 78 | 98.5% |

### EXAMPLE 20: EFFICACY OF C+ ON VETERINARIAN POST MORTEM EXAM ROOM FLOOR

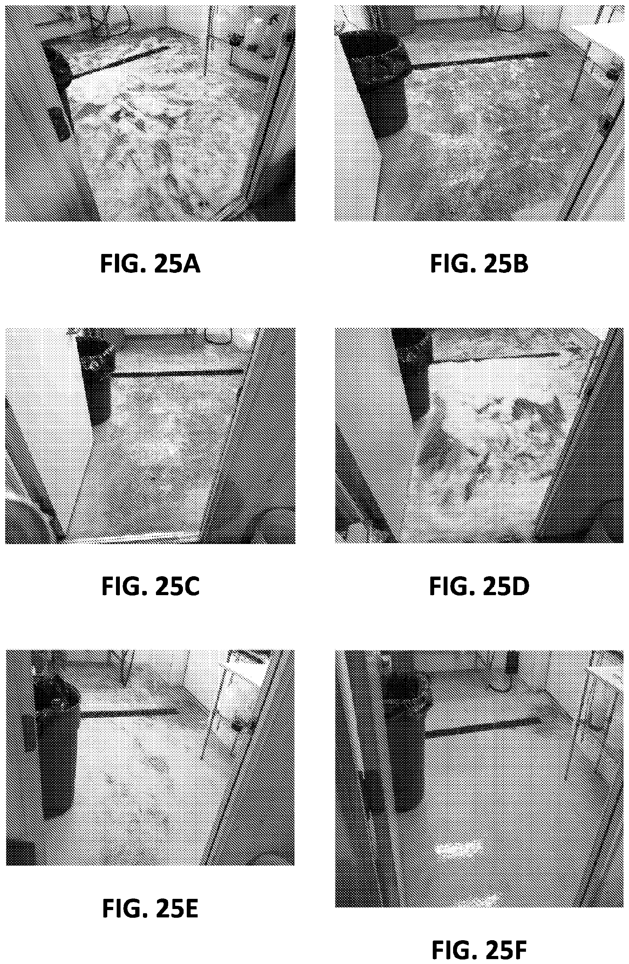

The efficacy of C+ at a dilution of 1:30 was tested on a veterinarian post-mortem exam room floor. Prior to the testing of C+, the procedure was to clean the floor by rinsing with water at line pressure from a garden hose followed by spraying the floor with Virkon Cleaner and Disinfectant (DuPont) and scrubbing the floor with a brush. The procedure used to test the efficacy of C+ was as follows. After each postmortem exam the floor was rinsed with water at normal line pressure from a garden hose. After rinsing the floor, C+ at a dilution of 1:30 was foamed onto the floor and allowed to remain there until the next time the room was used which was typically three days to one week. During the test there was no scrubbing or other mechanical agitation on the floor surface. All soil removal occurred due to chemical and biological means from the C+ ingredients. As shown in FIG. 25A to FIG. 25F. over a period of 2 weeks (during which the floor was foamed 4 times) the floor went from extremely dirty to acceptably clean. The odors that were in the room on Day 0, and the slippery floor, were completely eliminated by Day 14. FIG. 25A: day 0, foamed floor after rinsing. FIG. 25B: day 0, after rinsing and foaming floor. FIG. 25C: day 4, after rinsing, before foaming. FIG. 25D: day 4, foam on floor. FIG. 25E: day 8, after rinsing and foaming. FIG. 25F: day 14, after rinsing and before foaming.

### EXAMPLE 21: EFFICACY OF W+, M+, C+, AND HC+ AGAINST PATHOGENS ASSOCIATED WITH BRD

The efficacy of W+, M+, C+, and HC+ was tested against pathogens associated with BRD that is commonly found in Australian cattle feedlots. The following three groups of pathogens were tested using in vitro methods. 1) Primary viral organisms: bovine herpesvirus 1,2 (infectious bovine rhinotracheitis); bovine viral diarrhoea virus (pestivirus); parainfluenza virus (PI 3); and bovine respiratory syncytial virus. 2) Secondary involvement by bacteria present in the upper respiratory tract (nasopharynx ): Mannheimia haemolytica; Pasteurella multocida; and Histophilus somnus. 3) Tertiary Invaders: Actinomyces; Actinobacillus; and Mycoplasma. The following dilutions were tested: W+ at 1:10; M+ at 1:4; C+ at 1 :20; and HC+ at 1:8. Each of the four products prevented the growth of each of the three groups of pathogens. In other words, each product showed 100% inhibition of each of the pathogens tested.

### EXAMPLE 22: EFFICACY OF D+H TO TREAT HAIRY WART AND IMPROVE FOOT HEALTH

The efficacy of D+h to treat Hairy Wart was tested by pouring D+h diluted 1:400 into foot-bath troughs located at a herd dairy barn. The test procedure was as follows: replace copper-sulphate with D+h in the foot-bath troughs, and have the cows walk through the bath twice a day (at each milking).

With copper-sulphate, the usage rate is 25 pounds/100 gallons, and the solution is dumped and refilled after every 350 cows. With D+h, the usage rate is ¼ gallon/100 gallons (D+h 1:400), and the solution is diimpd and refilled after every 350 cows. The advantages of D+h are that it is safer (for animals and people) than formaldehyde, and safer for the environment than copper-sulphate which is contaminating farm lands and water-ways.

In the foot-bath application, copper-sulphate quickly becomes ineffective due to the chemical dilution when combined with cow manure (and cows almost immediately delicate when they walk into water). The D+h will, likewise, be diluted by the cow manure but the bacteria efficacy is not affected by the chemical change in the foot-bath solution. Additionally, the D+h does not have any negative effect on the environment and is complimentary to the rapid breakdown of manure into an effective fertilizer.

Significant improvement was observed in the reduction of hairy warts, by the reduction of new hairy wart formations on the feet, and by a reduction of odor from the feet. The overall health of the feet was also observed to be improved significantly.

### EXAMPLE 23: EFFECTIVENESS OF CO+, A1+, AND A SHAMPOO ON STAPHYLOCOCCUS INTERMEDIUS AND MALASSEZIA PACHYDERMATIS

The bacterium, Staphylococcus intermedius and the fungus, Malassezia pachydermatis, are both difficult to eradicate, and when present on the skin of dogs, these two organisms induce an acute skin infection.

Five specimens were obtained from dogs that were being treated at an animal hospital. Staphylococcus intermedius was present in two specimens, Malassezia pachydermatis was present in two specimens, and both organisms were present in one specimen. For each specimen, either one or both organisms were isolated and cultured. Each culture was plated and grown in appropriate conditions for 11 days.

Three products were tested on the five cultures during the 11-day period. A shampoo comprising 78.8% CO+, 5% C+, 10% Organic Glycerin, 5 % H2O, 1% Organic peppermint, and 0.2% Organic vanilla; CO+; and A1+. Each of the three products were tested at the following concentrations: neat, 1/2 dilution in water, and 1/4 dilution in water.

During the 11-day period, each of the plates was observed under inverted microscope, and the effect of the products on the organisms was studied. Control plates with each organism in the absence of any product were also cultured for 11 days.

CO+, A1+, and the shampoo were all effective against *Staphylococcus Intermedius* and *Malassezia pachydermatis.* All three products were effective either diluted or not. Up to 80% or 90% of the organisms were cleared.

## Claims

1. A composition for reducing a pathogen in an environment or for reducing a biofilm in an environment, comprising:
a stabilizer, at least one species of Bacillus, one or more surfactants, a protease or an amylase, and a disinfectant,
wherein the stabilizer is propylene glycol, glycerol, an oligomer or polymer of ethylene oxide, a methoxypolyethylene glycol, 1,2- dihydroxypropane, methyl glycol or trimethyl glycol;
the disinfectant is chlorine, chlorine dioxide, hydrogen peroxide or sodium hypochlorite; and
wherein the at least one species of Bacillus is Bacillus subtilis, Bacillus licheniformis, Bacillus pumilus, Bacillus coagulans, Bacillus amyloliquefaciens, Bacillus megaterium, or Bacillus mojavensis.

2. The composition of claim 1, wherein the one or more surfactant is a total volume of 0.5% to 1.0%, 1% to 10%, 10% to 20%, 20% to 30%, 30% to 40%, 40% to 50%, 50% to 60%, 60% to 70%, 70% to 80%, 80% to 90%, or 90% to 99% of the composition.

3. The composition of claim 1, wherein the stabilizer is a total volume of 0.1% to 0.2%, 0.2% to 0.3%, 0.3% to 0.4%, 0.4% to 0.5%, 0.5% to 0.6%, 0.6% to 0,7%, 0.7% to 0.8%, 0.8% to 0.9%, 0.9% to 1.0%, 1.0% to 2.0%, 2% to 5%, 5% to 10%, 10% to 20%, 20% to 30%, 30% to 40%, or 40% to 50% of the composition.

4. The composition of claim 1, wherein the protease is a total volume of 0.1% to 0.2%, 0.2% to 0.3%, 0.3% to 0.4%, 0.4% to 0.5%, 0.5% to 0.6%, 0.6% to 0,7%, 0.7% to 0.8%, 0.8% to 0.9%, 0.9% to 1.0%, 1.0% to 2.0%, 2.0% to 3.0%, 3.0% to 4.0%, 4.0% to 5.0%, 5% to 10%, 10% to 20%, 20% to 30%, 30% to 40%, or 40% to 50% of the composition.

5. The composition of claim 1, wherein the amylase is a total volume of 0.01 % to 0.02%, 0.02%to 0.03%, 0.03% to 0.04%, 0.04% to 0.05%, 0.05% to 0.06%, 0.06% to 0.07%, 0.07% to 0.08%, 0.08% to 0.09%, 0.09% to 0.1 %, 0.1 % to 1.0%, 1.0% to 2.0%, 2.0% to 3.0%, 3 .0% to 4.0%, 4.0% to 5.0%, 5% to 10%, 10% to 20%, 20% to 30%, 30% to 40%, or 40% to 50% of the composition.

6. The composition of claim 1, wherein the at least one species of Bacillus is a total volume of 0.01% to 0.02%, 0.02% to 0.03%, 0.03% to 0.04%, 0.04% to 0.05%, 0.05% to 0.06%, 0.06% to 0.07%, 0.07% to 0.08%, 0.08% to 0.09%, 0.09% to 1.0%, 1.0% to 2.0%, 2.0% to 3.0%, 3.0% to 4.0%, 4.0% to 5.0%, 5.0% to 6.0%, 6.0% to 7.0%, 7.0% to 8.0%, 8.0% to 9.0%, 9.0% to 10.0%, 10.0% to 15.0%, 15.0% to 20.0%, or 20.0% to 25.0% of the composition.

7. The composition of claim 1, wherein the surfactant is a soap, a detergent, a wetting agent, an emulsifier, a foaming agent, or a dispersant.

8. The composition of claim 1, wherein the composition is a) present on a wipe, a sponge, or a cloth; or b) a spray, a mist, a fog, a colloidal suspension of particles, a semi-solid form, or an aerosol spray; or c) a foam.

9. A method of reducing a pathogen in an environment, comprising: treating the environment with a composition of claim 8, wherein the environment is a surface selected from the group consisting of plastic, wood, ceramic, glass, stainless steel, steel, cast iron, plaster, paint, other metals, and cement; air; air space; or an aqueous solution.

10. A method of reducing a biofilm in an environment, comprising: treating the environment with a composition of claim 8, wherein the environment is a surface selected from the group consisting of plastic, wood, ceramic, glass, stainless steel, steel, cast iron, plaster, paint, other metals, and cement; air; air space; or an aqueous solution.

## Patentansprüche

1. Zusammensetzung zum Reduzieren eines Pathogens in einer Umgebung oder zum Reduzieren eines Biofilms in einer Umgebung, umfassend:
einen Stabilisator, mindestens eine Spezies von *Bacillus,* ein oder mehrere Tensid(e), eine Protease oder eine Amylase und ein Desinfektionsmittel,
wobei der Stabilisator Propylenglycol, Glycerin, ein Oligomer oder Polymer von Ethylenoxid, ein Methoxypolyethylenglycol, 1,2-Dihydroxypropan, Methylglycol oder Trimethylglycol ist;
das Desinfektionsmittel Chlor, Chlordioxid, Wasserstoffperoxid oder Natriumhypochlorit ist; und
wobei die mindestens eine Spezies von *Bacillus Bacillus subtilis, Bacillus licheniformis, Bacillus pumilus, Bacillus coagulans, Bacillus amyloliquefaciens, Bacillus megaterium* oder *Bacillus mojavensis* ist.

2. Zusammensetzung nach Anspruch 1, wobei das eine oder die mehreren Tensid(e) ein Gesamtvolumen von 0,5% bis 1,0%, 1% bis 10%, 10% bis 20%, 20% bis 30%, 30% bis 40%, 40% bis 50%, 50% bis 60%, 60% bis 70%, 70% bis 80%, 80% bis 90% oder 90% bis 99% der Zusammensetzung hat.

3. Zusammensetzung nach Anspruch 1, wobei der Stabilisator ein Gesamtvolumen von 0,1% bis 0,2%, 0,2% bis 0,3%, 0,3% bis 0,4%, 0,4% bis 0,5%, 0,5% bis 0,6%, 0,6% bis 0,7%, 0,7% bis 0,8%, 0,8% bis 0,9%, 0,9% bis 1,0%, 1,0% bis 2,0%, 2% bis 5%, 5% bis 10%, 10% bis 20%, 20% bis 30%, 30% bis 40% oder 40% bis 50% der Zusammensetzung hat.

4. Zusammensetzung nach Anspruch 1, wobei die Protease ein Gesamtvolumen von 0,1% bis 0,2%, 0,2% bis 0,3%, 0,3% bis 0,4%, 0,4% bis 0,5%, 0,5% bis 0,6%, 0,6% bis 0,7%, 0,7% bis 0,8%, 0,8% bis 0,9%, 0,9% bis 1,0%, 1,0% bis 2,0%, 2,0% bis 3,0%, 3,0% bis 4,0%, 4,0% bis 5,0%, 5% bis 10%, 10% bis 20%, 20% bis 30%, 30% bis 40% oder 40% bis 50% der Zusammensetzung hat.

5. Zusammensetzung nach Anspruch 1, wobei die Amylase ein Gesamtvolumen von 0,01% bis 0,02%, 0,02% bis 0,03%, 0,03% bis 0,04%, 0,04% bis 0,05%, 0,05% bis 0,06%, 0,06% bis 0,07%, 0,07% bis 0,08%, 0,08% bis 0,09%, 0,09% bis 0,1%, 0,1% bis 1,0%, 1,0% bis 2,0%, 2,0% bis 3,0%, 3,0% bis 4,0%, 4,0% bis 5,0%, 5% bis 10%, 10% bis 20%, 20% bis 30%, 30% bis 40% oder 40% bis 50% der Zusammensetzung hat.

6. Zusammensetzung nach Anspruch 1, wobei die mindestens eine Spezies von *Bacillus* ein Gesamtvolumen von 0,01% bis 0,02%, 0,02% bis 0,03%, 0,03% bis 0,04%, 0,04% bis 0,05%, 0,05% bis 0,06%, 0,06% bis 0,07%, 0,07% bis 0,08%, 0,08% bis 0,09%, 0,09% bis 1,0%, 1,0% bis 2,0%, 2,0% bis 3,0%, 3,0% bis 4,0%, 4,0% bis 5,0%, 5,0% bis 6,0%, 6,0% bis 7,0%, 7,0% bis 8,0%, 8,0% bis 9,0%, 9,0% bis 10,0%, 10,0% bis 15,0%, 15,0% bis 20,0% oder 20,0% bis 25,0% der Zusammensetzung hat.

7. Zusammensetzung nach Anspruch 1, wobei das Tensid eine Seife, ein Detergens, ein Netzmittel, ein Emulgator, ein Schaummittel oder ein Dispersionsmittel ist.

8. Zusammensetzung nach Anspruch 1, wobei die Zusammensetzung a) auf einem Wischtuch, einem Schwamm oder einem Tuch vorhanden; oder b) ein Spray, ein Sprühregen, ein Nebel, eine kolloidale Suspension von Partikeln, eine semi-solide Form oder ein Aerosolspray; oder c) ein Schaum ist.

9. Verfahren zum Reduzieren eines Pathogens in einer Umgebung, umfassend: Behandeln der Umgebung mit einer Zusammensetzung nach Anspruch 8, wobei die Umgebung eine Oberfläche ist, die ausgewählt ist aus der Gruppe bestehend aus Plastik, Holz, Keramik, Glas, rostfreiem Stahl, Stahl, Gusseisen, Gips, Farbe, anderen Metallen und Zement; Luft, Luftraum oder einer wässrigen Lösung.

10. Verfahren zum Reduzieren eines Biofilms in einer Umgebung, umfassend: Behandeln der Umgebung mit einer Zusammensetzung nach Anspruch 8, wobei die Umgebung eine Oberfläche ist, die ausgewählt ist aus der Gruppe bestehend aus Plastik, Holz, Keramik, Glas, rostfreiem Stahl, Stahl, Gusseisen, Gips, Farbe, anderen Metallen und Zement; Luft, Luftraum oder einer wässrigen Lösung.

## Revendications

1. Composition destinée à réduire un pathogène dans un environnement ou réduire un biofilm dans un environnement, comprenant :
un stabilisant, au moins une espèce de Bacillus, un ou plusieurs tensioactifs, une protéase ou une amylase, et un désinfectant,
dans laquelle le stabilisant est le propylène glycol, le glycérol, un oligomère ou polymère d'oxyde d'éthylène, un méthoxypolyéthylène glycol, le 1,2-dihydroxypropane, le méthylglycol ou le triméthylglycol ;
le désinfectant est le chlore, le dioxyde de chlore, le peroxyde d'hydrogène ou l'hypochlorite de sodium ; et
dans laquelle la au moins une espèce de Bacillus est Bacillus subtilis, Bacillus licheniformis, Bacillus pumilus, Bacillus coagulans, Bacillus amyloliquefaciens, Bacillus megaterium ou Bacillus mojavensis.

2. Composition selon la revendication 1, dans laquelle les un ou plusieurs tensioactifs représentent un volume total de 0,5 % à 1,0 %, 1 % à 10 %, 10 % à 20 %, 20 % à 30 %, 30 % à 40 %, 40 % à 50 %, 50 % à 60 %, 60 % à 70 %, 70 % à 80 %, 80 % à 90 %, ou 90 % à 99 % de la composition.

3. Composition selon la revendication 1, dans laquelle le stabilisant représente un volume total de 0,1 % à 0,2 %, 0,2 % à 0,3 %, 0,3 % à 0,4 %, 0,4 % à 0,5 %, 0,5 % à 0,6 %, 0,6 %, à 0,7 %, 0,7 % à 0,8 %, 0,8 % à 0,9 %, 0,9 % à 10 %, 10 % à 2,0 %, 2 % à 5 %, 5 % à 10 %, 10 % à 20 %, 20 % à 30 %, 30 % à 40 %, ou 40 % à 50 % de la composition.

4. Composition selon la revendication 1, dans laquelle la protéase représente un volume total de 0,1 % à 0,2 %, 0,2 % à 0,3 %, 0,3 % à 0,4 %, 0,4 % à 0,5 %, 0,5 % à 0,6 %, 0,6 % à 0,7 %, 0,7 % à 0,8 %, 0,8 % à 0,9 %, 0,9 % à 10 %, 10 % à 2,0 %, 2,0 % à 3,0 %, 3,0 % à 4,0 %, 4,0 % à 5,0 %, 5 % à 10 %, 10 % à 20 %, 20 % à 30 %, 30 % à 40 %, or 40 % à 50 % de la composition.

5. Composition selon la revendication 1, dans laquelle l'amylase représente un volume total de 0,01 % à 0,02 %, 0,02 % à 0,03 %, 0,03 % à 0,04 %, 0,04 % à 0,05 %, 0,05 % à 0,06 %, 0,06 % à 0,07 %, 0,07 % à 0,08 %, 0,08 % à 0,09 %, 0,09 % à 0,1 %, 0,1 % à 10 %, 10 % à 2,0 %, 2,0 % à 3,0 %, 3,0 % à 4,0 %, 4,0 %, à 5,0 %, 5 % à 10 %, 10 % à 20 %, 20 % à 30 %, 30 % à 40 %, ou 40 % à 50 % de la composition.

6. Composition selon la revendication 1, dans laquelle la au moins une espèce de Bacillus représente un volume total de 0,01 % à 0,02 %, 0,02 % à 0,03 %, 0,03 % à 0,04 %, 0,04 % à 0,05 %, 0,05 % à 0,06 %, 0,06 % à 0,07 %, 0,07 % à 0,08 %, 0,08 % à 0,09 %, 0,09 % à 10 %, 10 % à 2,0 %, 2,0 % à 3,0 %, 3,0 % à 4,0 %, 4,0 % à 5,0 %, 5,0 % à 6,0 %, 6,0 % à 7,0 %, 7,0 % à 8,0 %, 8,0 % à 9,0 %, 9,0 % à 10,0 %, 10,0 % à 15,0 %, 15,0 % à 20,0 %, ou 20,0 % à 25,0 % de la composition.

7. Composition selon la revendication 1, dans laquelle le tensioactif est un savon, un détergent, un agent mouillant, un émulsifiant, un agent moussant, ou un dispersant.

8. Composition selon la revendication 1, la composition étant a) présente sur une lingette, une éponge, ou un chiffon ; ou b) une pulvérisation, une brume, un brouillard, une suspension colloïdale de particules, une forme semi-solide, ou une pulvérisation d'aérosol ; ou c) une mousse.

9. Méthode de réduction d'un pathogène dans un environnement, comprenant : le traitement de l'environnement avec une composition selon la revendication 8, dans laquelle l'environnement est une surface choisie dans le groupe consistant en plastique, bois, céramique, verre, acier inoxydable, acier, fonte, plâtre, peinture, autres métaux, et ciment ; air ; espace d'air ; ou une solution aqueuse.

10. Méthode de réduction d'un biofilm dans un environnement, comprenant : le traitement de l'environnement avec une composition selon la revendication 8, dans laquelle l'environnement est une surface choisie dans le groupe consistant en plastique, bois, céramique, verre, acier inoxydable, acier, fonte, plâtre, peinture, autres métaux, et ciment ; air ; espace d'air ; ou une solution aqueuse.
